# EUROPÄISCHE PATENTANMELDUNG

(11) **EP 1 647 267 A1**
(43) Veröffentlichungstag der Anmeldung: **19.04.2006**
(21) Anmeldenummer: 05021920.3
(22) Anmeldetag: 07.10.2005
(51) Int. Cl.: A61K 8/81, A61Q 1/10, A61Q 5/00, A61Q 17/04, A61Q 19/00

(54) **Kosmetische, pharmazeutische und dermatologische Mittel**

(30) Priorität: 15.10.2004 DE 102004050239
(71) Anmelder: Clariant Produkte (Deutschland) GmbH, 65929 Frankfurt am Main (DE)
(72) Erfinder: Milbradt, Robert, Dr., 65191 Wiesbaden (DE); Stelter, Wibke, Dr., 61184 Großkarben (DE); Hornung, Michael, 65929 Frankfurt (DE); Lo Vasco, Sebastiano, 61197 Florstadt (DE)

(57) **Zusammenfassung**

Es werden kosmetische, dermatologische und/oder pharmazeutische Mittel beschrieben, die dadurch gekennzeichnet sind, dass sie mindestens ein Copolymer A, enthaltend
a) 1 bis 50 Gew.-% an Struktureinheiten hervorgegangen aus N-Vinylcaprolactam,
b) 49,99 bis 98,99 Gew.-% an wiederkehrenden Struktureinheiten der Formel (1) 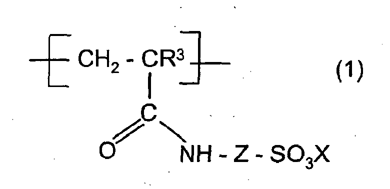 worin
   - R³: Wasserstoff, Methyl oder Ethyl,
   - Z: C₁-C₈-Alkylen und
   - X⁺: Li⁺, Na⁺, K⁺, Mg²⁺/2, Ca²⁺/2, Al³⁺/3, NH₄⁺, Monoalkylammonium, Dialkylammonium, Trialkylammonium oder Tetraalkylammonium, wobei es sich bei den Alkylsubstituenten der Ammmoniumionen unabhängig voneinander um (C₁-C₂₂)-Alkylreste oder (C₂-C₁₀)-Hydroxyalkylreste handelt, oder ein- bis dreifach ethoxylierte Ammoniumverbindungen mit gleichem oder unterschiedlichem Ethoxylierungsgrad bedeutet,
   und wobei auch mehrere unterschiedliche Struktureinheiten der Formel (1) in dem Copolymer enthalten sein können, und
c) 0,01 bis 8 Gew.-%, vorzugsweise 0,01 bis 5 Gew.-% an vernetzenden Strukturen, die aus Monomeren mit mindestens zwei olefinischen Doppelbindungen hervorgegangen sind,
enthalten.

## Beschreibung

Die vorliegende Erfindung betrifft insbesondere kosmetische, pharmazeutische und dermatologische Mittel enthaltend kammförmige Copolymere aus den Monomeren der Acryloyldimethyltaurinsäure und/oder deren Salzen und N-Vinylcaprolactam.

Verbraucherwünsche und Rheologie kosmetischer Produkte sind eng miteinander verknüpft. So wird. z.B. das visuelle Erscheinungsbild einer Creme oder Lotion durch die Viskosität beeinflusst. Die sensorischen Eigenschaften, wie Konsistenz oder Verteilbarkeit bestimmen das individuelle Profil eines Kosmetikproduktes. Auch die Effektivität von Wirksubstanzen (z.B. Sonnenschutzfilter) und auch die Lagerstabilität der Formulierung stehen in engem Zusammenhang mit den rheologischen Eigenschaften der Produkte.

Im kosmetischen Bereich kommt Polyelektrolyten als Verdicker und Gelbildner eine tragende Rolle zu. Stand der Technik sind insbesondere Verdicker auf Basis der Poly(meth)acrylsäure und deren wasserlöslichen Copolymeren. Die Vielfalt der möglichen Strukturen und die damit verbundenen vielfältigen Anwendungsmöglichkeiten drücken sich nicht zuletzt in einer Vielzahl von Patenten aus, die seit Mitte der 70ger Jahre weltweit angemeldet wurden.

Ein wesentlicher Nachteil der Verdicker auf Basis von Poly(meth)acrylsäure ist die starke pH-Abhängigkeit der Verdickungsleistung. So wird im allgemeinen eine hinreichend hohe Viskosität nur dann aufgebaut, wenn der pH-Wert der Formulierung oberhalb von pH 6 eingestellt wird, d.h. die Poly(meth)acrylsäure in neutralisierter Form vorliegt. Ferner sind die entsprechenden Mittel empfindlich gegenüber UV-Strahlung und auch Scherung und vermitteln zudem auf der Haut ein klebriges Gefühl. Ebenso ist die Handhabung derartiger Verdicker problematisch. Da die Verdicker im allgemeinen in saurer Form vorliegen, bedarf es bei der Formulierung eines zusätzlichen Neutralisationsschrittes.

In den 90iger Jahren wurden neuartige Verdicker auf Basis:vernetzter und neutralisierter Polyacryloyldimethyltaurate in den Markt eingeführt (EP 0 815 828, EP 0 815 844, EP 0 815 845 und EP 0 850 642). In EP 1 028 129 und EP 1 116 733 werden Copolymere aus Acryloyldimethyltaurinsäure und/oder deren Salzen und linearen N-Vinylcarbonsäureamiden bzw. Copolymere aus Acryloyldimethyltaurinsäure und/oder deren Salzen und linearen N-Vinylcarbonsäureamiden und cyclischen Vinylcarbonsäurenamiden (Aristoflex® AVC, Clariant GmbH) als Verdicker beschrieben. Diese auf Acryloyldimethyltaurat basierenden Verdickersysteme zeigen gute Eigenschaften in pH-Bereichen unterhalb von pH 6, aber auch eine hohe UV- und Scherstabilität, hervorragende viskoelastische Eigenschaften, eine leichte Verarbeitbarkeit und ein günstiges toxikologisches Profil.

Dennoch besteht großes Interesse daran, Verdickungsmittel zu entwickeln mit verbessertem Emulgier-, Dispergier- und Gelierungsvermögen bei möglichst geringen Einsatzkonzentrationen, die den Mitteln ein klares, ästhetisches Aussehen verleihen, gute Kompatibilität mit Zusatzstoffen zeigen, eine gute Hautsensorik bewirken und lagerstabil sind.

Überraschenderweise wurde gefunden, dass diese Aufgabe gelöst wird durch Copolymere A, enthaltend
a) 1 bis 50 Gew.-% an Struktureinheiten hervorgegangen aus N-Vinylcaprolactam,
b) 49,99 bis 98,99 Gew.-% an wiederkehrenden Struktureinheiten der Formel (1) 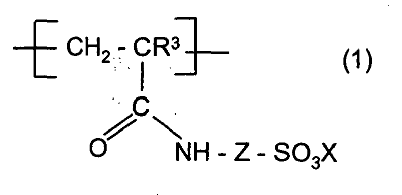 worin
   - R³: Wasserstoff, Methyl oder Ethyl,
   - Z: C₁-C₈-Alkylen und
   - X⁺: Li⁺,Na⁺, K⁺, Mg²⁺/2, Ca²⁺/2, Al³⁺/3, NH₄⁺, Monoalkylammonium, Dialkylammonium, Trialkylammonium oder Tetraalkylammonium, wobei es sich bei den Alkylsubstituenten der Ammmoniumionen unabhängig voneinander um (C₁-C₂₂)-Alkylreste oder (C₂-C₁₀)-Hydroxyalkylreste handelt, oder ein- bis dreifach ethoxylierte Ammoniumverbindungen mit gleichem oder unterschiedlichem Ethoxylierungsgrad bedeutet,
   und wobei auch mehrere unterschiedliche Struktureinheiten der Formel (1) in dem Copolymer enthalten sein können, und
c) 0,01 bis 8 Gew.-%, vorzugsweise 0,01 bis 5 Gew.-% an vernetzenden Strukturen; die aus Monomeren mit mindestens zwei olefinischen Doppelbindungen hervorgegangen sind.

Gegenstand der Erfindung sind daher kosmetische, dermatologische und/oder pharmazeutische Mittel, dadurch gekennzeichnet, dass sie mindestens ein Copolymer A, enthaltend
a) 1 bis 50 Gew.-% an Struktureinheiten hervorgegangen aus N-Vinylcaprolactam,
b) 49,99 bis 98,99 Gew.-% an wiederkehrenden Struktureinheiten der Formel (1) 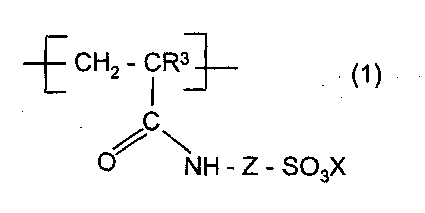 worin
   - R³: Wasserstoff, Methyl oder Ethyl,
   - Z: C₁-C₈-Alkylen und
   - X⁺: Li⁺, Na⁺, K⁺, Mg²⁺/2, Ca²⁺/2, Al³⁺/3, NH₄⁺, Monoalkylammonium, Dialkylammonium, Trialkylammonium oder Tetraalkylammonium, wobei es sich bei den Alkylsubstituenten der Ammmoniumionen unabhängig voneinander um (C₁-C₂₂)-Alkylreste oder (C₂-C₁₀)-Hydroxyalkylreste handelt, oder ein- bis dreifach ethoxylierte Ammoniumverbindungen mit gleichem oder unterschiedlichem Ethoxylierungsgrad bedeutet,
   und wobei auch mehrere unterschiedliche Struktureinheiten der Formel (1) in dem Copolymer enthalten sein können, und
c) 0,01 bis 8 Gew.-%, vorzugsweise 0,01 bis 5 Gew.-% an vernetzenden Strukturen, die aus Monomeren mit mindestens zwei olefinischen Doppelbindungen hervorgegangen sind,
enthalten.

Die Copolymerisate A zeigen ein verbessertes Emulsions- und Gelierungsvermögen bei im Vergleich zu Aristoflex® AVC reduzierten Einsatzkonzentrationen und haben auch in sauren Mitteln, insbesondere hydroxysäurehaltigen Zubereitungen, eine ausgezeichnete Verdickerleistung und bewirken eine gute Stabilisierung von Emulsionen auf ÖI-in-Wasser, Wasser-in-Öl und Wasser-in-Silikon Basis.
Die Copolymere A haben ein sehr gutes und schnelles Emulsionsvermögen, sind gut geeignet zur Viskositätseinstellung, auch elektrolythaltiger Mittel, verbessern die Lager- und Temperaturstabilität der Emulsionen bzw. Dispersionen und bewirken ein gutes Frischegefühl auf der Haut. Die Copolymere A zeigen ein ausgezeichnetes Dispergiervermögen fester Bestandteile in flüssigen oder pastenförmigen Mitteln und verbessern die Kompatibilität einzelner in kosmetischen und pharmazeutischen Mitteln üblicher Komponenten.

Sie sind daher vorteilhaft einsetzbar beispielsweise in dekorativen Mitteln, Sonnenschutzmitteln, Deodorantien, aber auch Haarfärbe-, -styling- und - pflegemitteln.
Sehr vorteilhaft lassen sich mit den Copolymeren A versprühbare, pumpbare und verschäumbare nicht-aerosolhaltige Gele und Schäume, insbesondere versprühbare Haargele und verschäumbare Sonnenschutzmittel mit optimiertem Sprühbild und optimierter Tröpfchengrößenverteilung herstellen.

Bevorzugte Copolymere A enthalten 2 bis 30, vorzugsweise 3 bis 15 Gew.-% an Struktureinheiten hervorgegangen aus N-Vinylcaprolactam, 69,5 bis 97,5, vorzugsweise 84,5 bis 96,5 Gew.-% an Struktureinheiten der allgemeinen Formel (1), insbesondere abgeleitet vom Ammoniumsalz der 2-Acrylamido-2-methyl-propan-sulfonsäure und 0,2 bis 4, vorzugsweise 0,5 bis 3 Gew.-% an vernetzenden Strukturen, die aus Monomeren mit mindestens zwei olefinischen Doppelbindungen hervorgegangen sind.

Vernetzende Strukturen, die aus Monomeren mit mindestens zwei olefinischen Doppelbindungen hervorgegangen sind, leiten sich vorzugsweise ab von Acryl- oder Methacrylsäureallylestern, Dipropylenglykol-diallylether, Polyglykoldiallylether, Triethylen-glykoldivinylether, Hydrochinon-diallylether, Tetraallyloxyethan oder anderen Allyl- oder Vinylethern multifunktioneller Alkohole, Tetraethylenglykol-diacrylat, Triallylamin, Trimethylolpropandiallylether, Methylen-bis-acrylamid, Trimethylolpropantriacrylat, Trimethylolpropantrimethacrylat oder Divinylbenzol.

In einer bevorzugten Ausführungsform leiten sich die vernetzenden Strukturen ab von Monomeren der allgemeinen Formel (2) 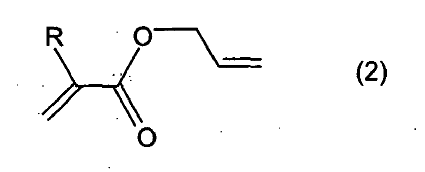 worin R Wasserstoff, Methyl oder Ethyl bedeutet. In einer weiteren bevorzugten Ausführungsform handelt es sich bei den vernetzenden Strukturen um Strukturen hervorgegangen aus Trimethylolpropantriacrylat gemäß der Formel (3)

H₅C₂―C[CH₂OOC-CH=CH₂]₃ (3)

Die Herstellung der Copolymere A erfolgt in der Weise, dass man die entsprechenden Monomeren a) und b) in einem protischen Lösungsmittel löst oder dispergiert, zu dieser Lösung oder Dispersion einen oder mehrere Vernetzer mit mindestens zwei olefinischen Doppelbindungen zugibt und die Polymerisation in an sich bekannter Weise durch Zugabe einer radikalbildenden Verbindung startet.

Bevorzugt wird das Ammoniumsalz der Acrylamidopropylsulfonsäure einpolymerisiert. Anstelle dieses Ammoniumsalzes kann man auch die freie Acrylamidopropylsulfonsäure einsetzen und vor der Zugabe der restlichen Monomere aus der freien Säure durch Einleiten von Ammoniak das Ammoniumsalz erzeugen.

In einer weiteren bevorzugten Ausführungsform bestehen die Copolymere A im Wesentlichen aus den Komponenten a),b) und c).

In einer weiteren bevorzugten Ausführungsform enthalten die erfindungsgemäßen Mittel, bezogen auf das Gesamtgewicht der Mittel, 0,01 bis 10 Gew.-%, vorzugsweise 0,1 bis 5 Gew.-%, besonders bevorzugt 0,5 bis 3 Gew.-% der Copolymere A.

Die Polymerisationsreaktion erfolgt vorzugsweise in einem wasserlöslichen Alkohol oder einem Gemisch mehrerer Alkohole mit 1 bis 6 C-Atomen, vorzugsweise in tert.-Butanol. Der Wassergehalt des Alkohols oder des Gemisches mehrerer Alkohole darf 10 Gew.-% nicht überschreiten, da sonst im Verlauf der Polymerisation Klumpenbildung auftreten kann. Konkret hat die Wahl der Art und der Menge des Lösungsmittels so zu erfolgen, dass das Salz der Acrylamidoalkylsulfonsäure der Formel (1), insbesondere der 2-Acrylamido-2-methyl-propan-sulfonsäure darin weitgehend löslich oder dispergierbar ist. Unter weitgehend löslich oder dispergierbar ist zu verstehen, dass sich auch nach Abstellen des Rührwerks kein festes Material aus der Lösung oder Dispersion absetzt. Das im Verlaufe der Reaktion entstehende Polymerisat soll hingegen in dem gewählten Lösungsmittel oder Lösungsmittelgemisch weitgehend unlöslich sein. Unter weitgehend unlöslich ist hierbei zu verstehen, dass im Verlauf der Polymerisation eine gut rührbare, breiige Polymerpaste entsteht, in der sich keine Klumpen oder Verklebungen bilden dürfen. Das durch Absaugen der Paste erhältliche Filtrat darf einen Feststoffgehalt von maximal 5 Gew.-% aufweisen. Sind die Copolymere in stärkerem Ausmaß im gewählten Lösungsmittel oder Lösungsmittelgemisch löslich, kann es beim Trocknen der Polymerisatpaste zu Verktümpungen kommen.

Die Polymerisationsreaktion selbst wird in an sich bekannter Weise durch radikalbildende Verbindungen wie Azoinitiatoren (z.B. Azo-bis-isobutyronitril), Peroxiden (z.B. Dilauroylperoxid) oder Persulfaten in einem geeigneten Temperaturintervall von 20 bis 120°C, vorzugsweise zwischen 40 und 80°C, ausgelöst und über einen Zeitraum von 30 Minuten bis mehrere Stunden fortgeführt.

Die Copolymerzusammensetzung lässt sich durch Variation des oben beschriebenen Einsatzverhältnisses der Monomere sowie des Anteils an Vernetzer variieren und so zur Erstellung eines maßgeschneiderten Eigenschaftsprofils verwenden. Durch verstärkten Einbau von Ammoniumsalzen der Acrylamidosulfonsäuren kann beispielsweise die verdickende Wirkung der Polymerisate verbessert werden, während durch Einbau von mehr N-Vinylcaprolactam die Elektrolytverträglichkeit der Polymerisate und deren Löslichkeit in nicht wässrigen Systemen verbessert werden kann.

In einer weiteren bevorzugten Ausführungsform haben die erfindungsgemäßen Mittel einen pH-Wert kleiner 6 und enthalten neben den Copolymeren A organische oder anorganische Säuren, vorzugsweise organische Säuren, besonders bevorzugt α-oder β-Hydroxysäuren.

Insbesondere bevorzugt sind α-Hydroxysäuren der Formel (4), 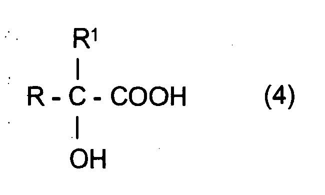 wobei R und R¹ unabhängig voneinander für H, F, Cl, Br, Alkyl-, Aralkyl- oder Arylgruppen mit gesättigten oder ungesättigten, linearen oder verzweigten Ketten, cyclischen Gruppen oder OH, CHO, COOH oder Alkoxygruppen mit 1 bis 9 Kohlenstoffatomen stehen können. Es kann die Säure und/oder das entsprechende Salz mit Alkali- oder Ammoniumgegenionen eingesetzt werden.

An sauren Komponenten in Betracht kommen Glykölsäure, Milchsäure, Methyllactonsäure, 2-Hydroxybutansäure, -pentan-, -hexan-, -heptan-, -octan-, nonan-, -decan-, -undecan-, -dodecansäure (Laurylsäure), α-Hydroxymyristylsäure, α-Hydroxypalmitylsäure, α-Hydroxystearylsäure, Arachidonsäure, 2-Phenyl-2-hydroxyethansäure, Zitronensäure, Weinsäure, Mandelsäure, Salicylsäure, Ascorbinsäure, Brenztraubensäure, 2,2-Diphenyl-2-hydroxyethansäure, 3-Phenyl-2-Hydroxypropansäure, 2-Phenyl-2-Methyl-2-Hydroxyethansäure, 2-(4'-Hydroxyphenyl)-2-Hydroxyethansäure, 2-(4'-Dichlorophenyl)-2-Hydroxyethansäure, 2-(3'-HydröXy-4'-Methoxyphenyl)-2-Hydroxyethansäure, 2-(4'-Hydroxy-3'-Methoxyphenyl)-2-Hydroxyethansäure, 3-(2'-Hydroxyphenyl)-2-Hydroxypropansäure, 3-(4'-Hydroxyphenyl)-2-Hydroxypropansäure, 2-(3', 4'-Dihydroxyphenyl)-2-Hydroxyethansäure, Fumarsäure, Retinoesäure, aliphatische und organische Sulfonsäuren, Benzoesäure, Kojisäure, Fruchtsäure, Äpfelsäure, Gluconsäure, Galacturonsäure, saure Pflanzen- und/oder Fruchtextrakte und deren Derivate.

In einer weiteren bevorzugten Ausführungsform enthalten die Mittel, bezogen auf das Gesamtgewicht der Mittel, 0,01 bis 20 Gew.-%, vorzugsweise 0,5 bis 10 Gew.-% und besonders bevorzugt 1 bis 5 Gew.-% an Hydroxysäuren, vorzugsweise α-Hydroxy-säuren, wobei diese auch teilweise als Salz vorliegen können. In einer besonders bevorzugten Ausführungsform enthalten diese Mittel, bezogen auf das Gesamtgewicht der Mittel, 0,01 bis 10 Gew.-% an Copolymer A.

Insbesondere bevorzugt enthalten die erfindungsgemäßen Mittel Glykolsäure, Milchsäure und/oder 2-Hydroxyoctansäure.

Die Hydroxysäure und das entsprechende Salz liegen vorzugsweise in einem molaren Verhältnis im Bereich von 1000 zu 1 bis 1 zu 1000, besonders bevorzugt 50 zu 1 bis 1 zu 50 vor.

Weitere bevorzugte Ausführungsformen der erfindungsgemäßen Mittel sind Flüssig-Flüssig-Dispersionen aus einer wässrigen und einer öligen Phase, insbesondere Wasser-in-Öl Emulsionen, Öl-in-Wasser Emulsionen, Öl-in-Wasser Mikroemulsionen und multiple Emulsionen, Gelcremes, sowie Fest-Flüssig-Dispersionen.

Die Herstellung der Emulsionen kann in bekannter Weise, d.h. beispielsweise durch Heiß-, Heiß/Kalt- bzw. PIT-Emulgierung erfolgen.

Die Copolymere A haben ein sehr gutes und schnelles Emulsionsvermögen, sind gut geeignet zur Viskositätseinstellung, verbessern die Lager- und Temperaturstabilität der Emulsionen bzw. Dispersionen und bewirken ein gutes Frischegefühl auf der Haut.

Die Copolymere A zeigen ein ausgezeichnetes Dispergiervermögen fester Bestandteile in flüssigen oder pastenförmigen Mitteln und verbessern die Kompatibilität einzelner Komponenten. Sie sind daher vorteilhaft einsetzbar beispielsweise in dekorativen Mitteln, Sonnenschutzmitteln und Deodorantien.

Sehr vorteilhaft lassen sich mit den Copolymeren A pumpbare und verschäumbare nicht-aerosolhaltige Gele und Schäume; insbesondere versprühbare Haargele und verschäumbare Sonnenschutzmittel herstellen.

In weiteren bevorzugten Ausführungsformen enthalten die erfindungsgemäßen Mittel Wasser, Öl, einen oder mehrere Emulgatoren und ein oder mehrere Copolymere A.

Im Fall, dass die erfindungsgemässen Mittel Emulsionen darstellen, kann die Ölphase vorteilhafterweise ausgewählt werden aus den Gruppen der Mineralöle, Mineralwachse, Öle, wie Triglyceride, Fette, Wachse und andere natürliche und synthetische Fettkörper, vorzugsweise Ester von Fettsäuren mit Alkoholen niedriger C-Zahl, z.B. mit Isopropanol, Propylenglycol oder Glycerin, oder Ester von Fettalkoholen mit Alkansäuren niedriger C-Zahl mit Fettsäuren, Alkylbenzoate.

Eine Klasse von erfindungsgemäß bevorzugten Ölen und Fetten sind die Triglyceride von linearen oder verzweigten, gesättigten oder ungesättigten, gegebenenfalls hydroxylierten C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Wainuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z.B. das Handelsprodukt Myritol® 318. Auch gehärtete Triglyceride sind erfindungsgemäß bevorzugt.
Auch Öle tierischen Ursprungs, beispielweise Rindertalg, Perhydrosqualen, Lanolin können eingesetzt werden.

Eine weitere Klasse von erfindungsgemäß bevorzugten Ölen und Fetten sind die Benzoesäureester von linearen oder verzweigten C₈₋₂₂-Alkanolen, z.B. die Handelsprodukte Finsolv® SB (Isostearylbenzoat), Finsolv® TN (C₁₂-C₁₅-Alkylbenzoat) und Finsolv® EB (Ethylhexylbenzoat).

Eine weitere Klasse von erfindungsgemäß bevorzugten Ölen und Fetten sind die Dialkylether mit insgesamt 12 bis 36 Kohlenstoffatomen, insbesondere 12 bis 24C-Atomen, wie z.B. Di-n-octylether (Cetiol® OE), Di-n-decylether, Di-n-nonylether, Di-n-undecylether, Di-n-dodecylether, n-Hexyl-n-octylether, n-Octyl-n-decytether, n-Decyln-undecylether, n-Undecyl-n-dodecylether und n-Hexyl-n-Undecylether sowie Di-tert-butylether, Di-iso-pentylether, Di-3-ethyldecylether, tert-Butyl-n-octylether, iso-Pentyl-n-octylether und 2-Methyl-pentyl-n-octylether.

Eine weitere Klasse von erfindungsgemäß bevorzugten Ölen und Fetten sind Kohlenwasserstofföie zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, hydrierte Polyisobutylene, Docosane, Hexadecan, Isohexadecan, Paraffinöle, Paraffiflwachse, Isoparaffinöle, z.B: die Handelsprodukte der Permethy® -Serie, Squalan, Squalen, synthetische Kohienwasserstoffe wie Polyisobuten und alicyclische Kohlenwasserstoffe, z.B. das Handelsprodukt 1,3-Di-(2-ethyl-hexyl)-cyclohexan (Cetiol® S), Ozokerit, Mikrowachse und Ceresin.

Ebenso in Betracht kommen verzweigte gesättigte oder ungesättigte Fettalkohole mit 6 - 30 Kohlenstoffatomen, z.B. Isostearylalkohol, sowie Guerbetalkohole.

Eine weitere Klasse von erfindungsgemäß bevorzugten Ölen und Fetten sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Hydroxycarbonsäureester sind Ester β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z.B. unter dem Handelsnamen Cosmacol® der EniChem, Augusta Industriale.

Eine weitere Klasse von erfindungsgemäß bevorzugten Ölen und Fetten sind Dicarbonsäureester von linearen oder verzweigten C₂-C₁₀-Alkanolen, wie Di-n-butyladipat (Cetiol® B), Di-(2-ethylhexyl)-adipat, Di-(2-ethylhexyl)-succinat und Diisotridecylacelaat sowie Diolester wie Ethylenglycol-dioleat, Ethylenglycol-diisotridecanoat, Propylenglycol-di(2-ethylhexanoat), Propylenglycol-di-isostearat, Propylenglycol-di-pelargonat, Butandiol-di-isostearat und Neopentylglycoldicaprylat.

Ebenso bevorzugte Öle und Fette sind symmetrische, unsymmetrische oder cyclische Ester der Kohfensäure mit Fettalkoholen, Glycerincarbonat oder Dicaprylylcarbonat (Cetiol® CC).

Eine weitere Klasse von erfindungsgemäß bevorzugten Ölen und Fetten sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. In einer weiteren bevorzugten Ausführungsform der Erfindung liegen die erfindungsgemäßen Mittel in Form einer Wasser-in-Silicon Emulsion vor und enthalten Wasser, Silikon, einen oder mehrere Emulgatoren und ein oder mehrere Copolymere A.

An Silikonölen bzw -wachsen stehen vorzugsweise zur Verfügung Dimethylpolysiloxane und Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, sowie die unter SilCare® Silicone 41M65, SilCare® Silicone 41 M70, SilCare® Silicone 41 M80 (Clariant GmbH) erhältlichen Dimethicone Stearyldimethylpolysiloxan, C₂₀-C₂₄-Alkyl-dimethylpolysiloxan, C₂₄-C₂₈-Alkyl-dimethylpolysiloxan, aber auch die unter SilCare® Silicone 41M40, SilCare® Silicone 41 M50 (Clariant GmbH) erhältlichen Methicone, weiterhin Trimethylsiloxysilicate [(CH₂)₃SiO)_{1/2}]ₓ[SiO₂]y, wobei x für eine Zahl von 1 bis 500 und y für eine Zahl von 1 bis 500 steht,Dimethiconole R₃SiO[R₂SiO]ₓSiR₂OH und HOR₂SiO[R₂SiO]ₓSiR₂OH, wobei R für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-; fettsäure-, alkohol-, polyether-, epoxy-, fluor- und/oder alkylmodifizierte Silikonverbindungen, sowie Polyethersiloxan-Copolymere.

Als Emulgator kommen nichtionische, anionische und ampholytische oberflächenaktive Mittel in Betracht.

Als nichtionogene Emulgatoren stehen zur Verfügung Anlagerungsprodukte von 0 bis 30 Mol Ethylenoxid und/oder 0 bis 5 Mol Propylenoxid an lineare Fettalkohole mit 6 bis 30 C-Atomen, bevorzugt 10 bis 22 C-Atomen, und ganz besonders bevorzugt 14 bis 22 C-Atomen, gegebenenfalls hydroxyliert. Einsetzbar sind beispielsweise Octanol (Caprylalkohol), Octenol, Octadienol, Decanol (Caprinalkohol), Decenol, Decadienol, Dodecenol (Laurylalkohol), Dodecanol, Dodecadienol, Ricinolalkohol, Myristylalkohol, Cetylalkohol, Stearylalkohol, Cetearylalkohol, Oleylalkohol, Linoleylalkohol, Linolenylalkohol, Arachidylalkohol, Behenylalkohol. Einsetzbar sind auch Fettalkoholschnitte, die durch Reduktion natürlich vorkommender Triglyceride, wie Rindertalg, Palmöl, Erdnussöl, Rüböl, Baumwollsaatöl, Sojaöl, Sonnenblumenöl und Leinöl oder aus Umesterungsprodukten mit entsprechenden Alkoholen entstehenden Fettsäureestern erzeugt werden und somit ein Gemisch von unterschiedlichen Fettalkoholen darstellen. Solche Substanzen sind beispielsweise unter der Bezeichnung Stenol® , z.B. Stenol® 1618 oder Lanette® , z.B. Lanette® ○ und Lanette® 22 oder Lorol® , z.B. Lorol® C18 im Handel erhältlich. Geeignet sind auch Wollwachsfette.
Besonders bevorzugt sind Cetylalkohol, Stearylalkohol, Cetearylalkohol, Arachidylalkohol und Behenylalkohol.
Eine weitere Klasse von erfindungsgemäß bevorzugten Emulgatoren sind Anlagerungsprodukte von 0 bis 30 Mol Ethylenoxid und/oder 0 bis 5 Mol Propylenoxid an lineare und/oder verzweigte, gesättigte und/oder ungesättigte Fettsäuren mit 6 bis 30 C-Atomen, bevorzugt 10 bis 22 Kohlenstoffatomen. Zu nennen sind Isostearinsäure, wie die Handelsprodukte Emersol® 871 und Emersol® 875, Isopalmitinsäuren wie Edenor® IP95, sowie alle weiteren unter der Handelsbezeichnung Edenor® (Cognis) käuflichen Fettsäuren. Weitere typische Beispiele für solche Fettsäuren sind Capronsäure, Caprylsäure, 2-Ethylhexansäure, Caprinsäure, Laurinsäure, lsotridecansäure, Myristinsäure, Palmitinsäure, Palmitoleinsäure, Stearinsäure, lsostearinsäure, Ölsäure, Elaidinsäure, Petroselinsäure, Linolsäure, Linolensäure, Elaeosterinsäure, Arachinsäure, Gadoleinsäure, Behensäure, Erucasäure und Dimere von ungesättigten Fettsäuren, sowie deren technische Mischungen. Besonders bevorzugt sind Fettsäureschnitte aus Cocosöl oder Palmöl; insbesondere bevorzugt ist Stearinsäure.
Üblicherweise werden die Fettsäuren mit einem basischen Mittel, z.B. NaOH, neutralisiert und beispielsweise in Form ihrer Natrium-, Kalium-, Ammonium-, Calcium-, Magnesium- und Zink-Salze verwendet.

Eine weitere Klasse von einsetzbaren Emulgatoren sind Ester von gewünschtenfalls alkylierten Zuckern mit C₆-C₃₀-Fettsäuren. Als Zucker können beliebige Mono- oder Oligosaccharide eingesetzt werden. Üblicherweise werden Monosaccharide mit 5 oder 6 Kohlenstoffatomen eingesetzt, beispielsweise Ribose, Xylose, Lyxose, Altose, Glucose, Fructose, Galactose, Arabinose, Altrose, Mannose, Gulose, Idose, Talose, sowie die Desoxyzucker Rhamnose und Fucose. Auch Zucker mit 4 Kohlenstoffatomen können eingesetzt werden, z.B. Erythrose und Threose. Erfindungsgemäß geeignete Oligosaccharide sind aus zwei bis 10 Monosaccharideinheiten zusammengesetzt, z.B. Sucrose (Saccharose), Lactose oder Trehalose. Bevorzugte Zuckerbausteine sind die Monosaccharide Glucose, Fructose, Galactose, Arabinose und das Disaccharid Sucrose; Glucose und Sucrose sind besonders bevorzugt. Die Zucker können partiell mit Methyl, Ethyl-, Propyl-, Isopropyl- oder Butylgruppen verethert sein, z.B. Methylglucosid, Ethylglucosid oder Butylglucosid. Zur Veresterung können alle C₆-C₃₀-Fettsäuren und deren Mischungen verwendet werden, die vorstehend genannt wurden. Geeignet sind prinzipiell einfach und mehrfach veresterte Zucker; bevorzugt sind die Mono-, Sesqui- und Diester, beispielsweise Sucrosemonostearat, Sucrosedistearat, Sucrosemonococoat, Sucrosedicocoat, Methylglucosidmonostearat, Methylglucosidsesquistearat, Methylglucosidisostearat, Ethylglucosidmonolaurat, Ethylglucosiddilaurat, Ethylglucosidmonococoat, Ethylglucosiddicocoat und Butylglucosidmonococoat.

Eine weitere Klasse geeigneter Emulgatoren sind C₈-C₂₂-Alkylmono- und -oligoglycoside, Alkylmono- und ―oligoglycoside, entsprechend der allgemeinen Formel RO-(Z)ₓ, wobei R für eine C₈-C₂₂-Alkylgruppe, Z für Zucker und x für die Anzahl der Zuckereinheiten stehen. Die erfindungsgemäß verwendbaren Alkylmono- und -oligoglycoside können lediglich einen bestimmten Alkylrest R enthalten. Besonders bevorzugt sind solche Alkylmono- und -oligoglycoside, bei denen R im wesentlichen aus C₈- und C₁₀-Alkylgruppen, im wesentlichen aus C₁₂- und C₁₄₋Alkylgruppen, im wesentlichen aus C₈- bis C₁₆-Alkylgruppen oder im wesentlichen aus C₁₂- bis C₁₆-Alkylgruppen besteht. Als Zuckerbaustein Z können beliebige Mono- oder Oligosaccharide, wie sie vorstehend genannt wurden, eingesetzt werden. Bevorzugte Zuckerbausteine sind Glucose, Fructose, Galactose, Arabinose und Sucrose, wobei Glucose besonders bevorzugt ist. Die erfindungsgemäß verwendbaren Alkylmono- und -oligoglycoside enthalten im Schnitt 1,1 - 5, bevorzugt 1,1 -2,0 und besonders bevorzugt 1,1 - 1,8 Zuckereinheiten. Auch die alkoxylierten Homologen der genannten Alkylmono- und -oligoglycoside können erfindungsgemäß eingesetzt werden. Diese Homologen können durchschnittlich bis zu 10 Ethylenoxid- und/oder Propylenoxideinheiten pro Alkylglycosideinheit enthalten. Geeignet sind beispielsweise Cocoylglucosid, Decylglucosid, Laurylglucosid, Cetearylglucosid und Arachidylglucosid.
Besonders bevorzugt sind neben den genannten Alkylmono- und -oligoglucosiden auch die Gemische aus Alkylmono- und -oligoglucosiden und Fettalkoholen, z.B. die im Handel erhältlichen Produkte Montanov® 68 und Montanov® 202.

Eine weitere Klasse geeigneter Emulgatoren sind die Partialester von Propylenglycol, Glycerin und Sorbitan mit C₈-C₂₂-Fettsäuren. Zur Veresterung können alle C₈-C₂₂-Fettsäuren und deren Mischungen verwendet werden, die vorstehend bereits genannt wurden. Besonders geeignete Beispiele sind Propylenglycolmonostearat, Glycerinmonolaurat, Glycerinmonostearat, Glycerindistearat, Glycerinmonooleat, Sorbitanmonolaurat, Sorbitandilaurat, Sorbitanmonöstearat, Sorbitansesquistearat, Sorbitandistearat, Sorbitanmonoisostearat, Sorbitanmonooleat, Sorbitandioleat oder die Handelsprodukte Monomuls® 90-0, Monomuls® 90-L 12 und Cutina® MD. Diese Emulgatoren können durchschnittlich bis zu 10 Ethylenoxid- und/oder Propylenoxideinheiten pro Molekül enthalten.

Eine weitere bevorzugte Klasse an Emulgatoren sind Polyglycerine der Formel HO-CH₂-CHOH-CH₂[-O-CH₂-CHOH-CH₂]ₙ-O-CH₂-CHOH-CH₂OH mit n = 0-8 und deren Ester mit linearen und verzweigten C₈-C₂₂-Fettsäuren, die in der Alkylkette funktionelle Gruppen tragen können, bevorzugt Polyglyceryl-2-dipolyhydroxystearat (Handelsprodukt Dehymuls® PGPH) und Polyglyceryl-3-diisostearat (Handelsprodukt Lameforme TGI).

Eine weitere Klasse bevorzugter Emulgatoren sind Sterole (Sterine), insbesondere Cholesterol, Lanosterol, β-Sitosterol, Stigmasterol, Campesterol und Ergosterol sowie Mykosterole. Handelsübliche Sterol-Emulgatoren werden auf der Basis von Soja- oder Rapssterolen hergestellt. Erfindungsgemäß bevorzugt ist der Einsatz von Sterolen, die 5 - 10 Ethylenoxideinheiten pro Molekül enthalten. Geeignet sind z.B. die Handelsprodukte Generol® 122, Generol® 122 E 5, Generol® 122 E 10 und Generol® RE 10.

Ebenso bevorzugt einsetzbare Emulgatoren sind Phospholipide, vor allem die Phosphatidycholine oder Lecithine. Phospholipide sind Phosphorsäurediester, seltener -monoester, von zumeist linearen gesättigten und ungesättigten C₈-C₂₂₋Fettsäuren; bevorzugt ist Sojalecithin.

Eine weitere Klasse bevorzugter Emulgatoren sind die Veresterungsprodukte von Milchsäure oder Giykoisäure mit linearen oder verzweigten C₈-C₂₂-Fettsäüren sowie die Natrium-, Kalium-, Ammonium-, Caicium-, Magnesium- und Zink-Saize dieser Veresterungsprodukte.

Besonders bevorzugt sind Veresterungsprodukte der allgemeinen Formel (5) 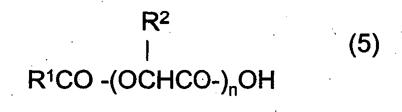 wobei R¹ einen linearen oder verzweigten gesättigten oder ungesättigten Alkylrest mit 5 bis 21 Kohlenstoffatomen und R² eine Methylgruppe oder ein Wasserstoffatom darstellen und n eine ganze Zahl von 1 - 4 ist.
Unter den Acylresten R¹CO- sind wiederum die Reste ausgewählt aus der Caproyl-, Capryloyl-, Caprinoyl-, Lauroyl-, Myristoyl-, Cetoyl-, Palmitoyl-, Stearoyl-, Isostearoyl- und der Oleyl-Gruppe bevorzugt. Besonders bevorzugt sind die Stearoyl- und die Isostearoyl-Gruppe. Der Rest R² ist vorzugsweise Methyl.
Der Oligomerisierungsgrad n ist vorzugsweise 1 oder 2.
Insbesondere bevorzugt ist die Verbindung Natriumstearoyl-2-lactylat.

Eine weitere Klasse bevorzugt eingesetzter Emulgatoren sind Phosphorsäuremono-, -di-, und ―triester von gesättigten oder ungesättigten linearen oder verzweigten Fettalkoholen mit 8 bis 30 Kohlenstoffatomen und ihre Ethylenoxidaddukte mit 1 - 10 Ethylenoxid-Gruppen pro Molekül Diese Alkyl-und Alkenylphosphate sind in der allgemeinen Formel (6) dargestellt 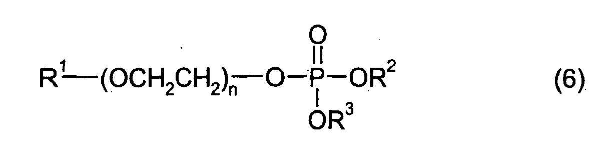 in der R¹ einen gesättigten oder ungesättigten, linearen oder verzweigten Kohlenwasserstoffrest mit 8 bis 30 Kohlenstoffatomen, R² und R³ unabhängig voneinander ein Wasserstoffatom, X oder einen Rest (CH₂CH₂O)ₙR¹, n Zahlen von 0 bis 10 und X ein Alkali- oder Erdalkalimetallkation oder ein Kation NR⁴R⁵R⁶R⁷, mit R⁴ bis R⁷unabhängig voneinander stehend für einen C₁-C₄-Kohlenwasserstoffrest, darstellen.
Die erfindungsgemäß bevorzugten Alkyl- und Alkenylphosphate weisen als Gruppe R¹ Alkylreste mit 12 - 18 Kohlenstoffatomen auf, die gesättigt oder ungesättigt sowie linear oder verzweigt sein können. Diese Gruppen R¹ sind insbesondere Lauryl, Myristyl, Cetyl, Palmityl, Stearyl, Isostearyl und Oleyl. Bevorzugte Werte für n sind entweder 0 oder Werte von 1-10, bevorzugt 2 - 5, besonders bevorzugt 3 - 4 (Alkyl- oder Alkenyletherphosphate). Weiterhin bevorzugt ist die Verwendung von Estergemischen aus Mono-, Di- und Triestern wobei der Anteil an Mono- und Diester gegenüber dem Triesteranteil überwiegt. Die Verwendung von reinen Triestern kann aber ebenfalls bevorzugt sein. Geeignete Handelsprodukte stammen z.B. aus der Hostaphat® -Serie (Clariant), z.B. Hostaphat® KW 340 D, Hostaphat® KO300 N, Hostaphat® KO380 und Hostaphat® KL 340.

Eine weitere Klasse von erfindungsgemäß bevorzugt eingesetzten Emulgatoren sind Acylglutamate der Formel (7) 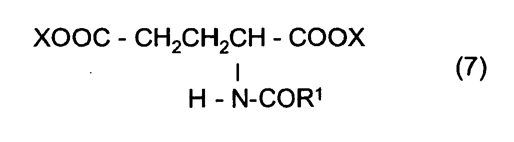 in der R¹CO für einen linearen oder verzweigten Acylrest mit 6 bis 22 Kohlenstoffatomen und 0, 1, 2 oder 3 Doppelbindungen und X für Wasserstoff, ein Alkali- oder Erdalkalimetallkation, für ein Ammonium, Alkylammonium, Alkanolammonium und/oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat.

Eine weitere Klasse von erfindungsgemäß bevorzugten Emulgatoren sind die Ester einer hydroxysubstituierten Di- oder Tricarbonsäure der allgemeinen Formel (8), 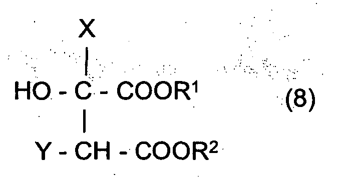 in der X H oder eine ―CH₂COOR-Gruppe ist, Y H oder ―OH ist unter der Bedingung, dass Y H ist, wenn X -CH₂COOR ist, R, R¹ und R² unabhängig voneinander ein Wasserstoffatom, ein Alkali- oder ein Erdalkalimetallkation, eine Ammoniumgruppe, das Kation einer ammonium-organischen Base oder einen Rest Z bedeuten, der von einer polyhydroxylierten organischen Verbindung stammt die aus der Gruppe der veretherten (C₆-C₁₈)-Alkylsaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, dass wenigstens eine der Gruppen R, R¹ oder R² ein Rest Z ist.

Eine weitere Klasse von erfindungsgemäß bevorzugten Emulgatoren sind die Ester des Sulfobernsteinsäuresalzes der allgemeinen Formel (9) 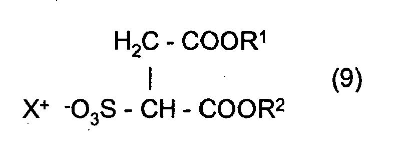 in der R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₈)-Hydroxyalkylpolyole mit 2 bis 16 Hydroxylresten ausgewählt ist, unter der Maßgabe, dass wenigstens eine der Gruppen R¹ oder R² ein Rest Z ist, und X⁺ ein Alkali- oder Erdalkalimetallkation, eine Ammoniumgruppe oder das Kation einer ammonium-organischen Base bedeutet.

Eine weitere Klasse von erfindungsgemäß bevorzugten Emulgatoren sind die Sulfobernsteinsäuremono- und ―dialkylester mit 8 bis 24 C-Atomen in der Alkylgruppe und Sulfobernsteinsäuremonoalkylpolyoxyester mit 8 bis 24 C-Atomen in der Alkylgruppe und 1 bis 6 Ethoxygruppen und ihre Alkali-, Erdalkalimetall- oder Ammoniumsalze.
Eine weitere Klasse von erfindungsgemäß bevorzugten Emulgatoren sind die Ester der Weinsäure und Zitronensäure mit Alkoholen, die Anlagerungsprodukte von etwa 2 bis 10 Molekülen Ethylenoxid und/oder Propylenoxid an Fettalkohole mit 8 bis 22 C-Atomen darstellen und ihre Alkali-, Erdalkalimetall- oder Ammoniumsalze.

Weitere erfindungsgemäß bevorzugte Emulgatoren sind Ethercarbonsäuren der Formel R-O-(CH₂-CH₂O)ₓ-CH₂-COOH, in der R eine lineare Alkylgruppe mit 8 bis 30 C-Atomen und x 0 oder 1 bis 10 ist, Acylsarcosinate mit einem linearen oder verzweigten Acylrest mit 6 bis 22 Kohlenstoffatomen und 0, 1, 2 oder 3 Doppelbindungen, Acyltaurate mit einem linearen oder verzweigten Acylrest mit 6 bis 22 Kohlenstoffatomen und 0, 1, 2 oder 3 Doppelbindungen sowie Acylisethionate mit einem linearen oder verzweigten Acylrest mit 6 bis 22 Kohlenstoffatomen und 0, 1, 2 oder 3 Doppelbindungen, sowie die Alkali-, Erdalkalimetall- oder Ammoniumsalze dieser Emulgatoren.

In den erfindungsgemäßen Mitteln können Gemische von Verbindungen aus mehreren dieser Substanzklassen enthalten sein.

In einer bevorzugten Ausführungsform liegen die erfindungsgemäßen Mittel als Emulsionen vom Typ Öl-in-Wasser, vorzugsweise als kosmetische oder dermatologische Emulsionen vom Typ Öl-in-Wasser, vor und enthalten bezogen auf das Gesamtgewicht der Mittel
a) bis zu 95 Gew.-%, vorzugsweise 60 bis 92 Gew.-%, besonders bevorzugt 70 bis 90 Gew.-%, insbesondere bevorzugt 75 bis 85 Gew.-% einer Wasserphase,
b) bis zu 40 Gew.-%, vorzugsweise 1 bis 40 Gew.-%, besonders bevorzugt 2 bis 25 Gew.-%, insbesondere bevorzugt 5 bis 20 Gew.-% einer Ölphase,
c) bis zu 15 Gew.-%, vorzugsweise 0,5 bis 12 Gew.-%, besonders bevorzugt 1 bis 8 Gew.-%, insbesondere bevorzugt 1 bis 5 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymer A.

In einer weiteren bevorzugten Ausführungsform liegen die erfindungsgemäßen Mittel als Gelcremes vom Typ Öl-in-Wasser, vorzugsweise als kosmetische oder dermatologische Gelcremes vom Typ Öl-in-Wasser, vor und enthalten bezogen auf das Gesamtgewicht der Mittel
a) bis zu 95 Gew.-%, vorzugsweise 50 bis 95 Gew.-%; besonders bevorzugt 70 bis 90 Gew.-%, insbesondere bevorzugt 75 bis 85 Gew.-% einer Wasserphase,
b) bis zu 30 Gew.-%, vorzugsweise 1 bis 30 Gew.-%, besonders bevorzugt 3 bis 25 Gew.-%, insbesondere bevorzugt 5 bis 15 Gew.-% einer Ölphase,
c) bis zu 5 Gew.-%, vorzugsweise 0,5 bis 5 Gew.-%, besonders bevorzugt 0,2 bis 4 Gew.-%, insbesondere bevorzugt 0,5 bis 3 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymer A.

In einer weiteren bevorzugten Ausführungsform liegen die erfindungsgemäßen Mittel als Emulsionen vom Typ Wasser-in-Öl, vorzugsweise als kosmetische oder dermatologische Emulsionen vom Typ Wasser-in-Öl, vor und enthalten bezogen auf das Gesamtgewicht der Mittel
a) bis zu 95 Gew.-%, vorzugsweise 40 bis 95 Gew.-%, besonders bevorzugt 50 bis 90 Gew.-%, insbesondere bevorzugt 60 bis 85 Gew.-% einer Wasserphase,
b) bis zu 60 Gew.-%, vorzugsweise 2 bis 60 Gew.-%, besonders bevorzugt 5 bis 40 Gew.-%, insbesondere bevorzugt 10 bis 30 Gew.-% einer Ölphase,
c) bis zu 20 Gew.-%, vorzugsweise 0,5 bis 20 Gew.-%, besonders bevorzugt 1 bis 15 Gew.-%, insbesondere bevorzugt 4 bis 12 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymer A.

In einer weiteren bevorzugten Ausführungsform liegen die erfindungsgemäßen Mittel als Wasser-in-Silikon-Emulsionen, vorzugsweise als kosmetische oder dermatologische Wasser-in-Silikon-Emulsionen, vor und enthalten bezogen auf das Gesamtgewicht der Mittel
a) bis zu 90 Gew:-%, vorzugsweise 20 bis 90 Gew.-%, besonders bevorzugt 40 bis 85 Gew.-%, insbesoridere bevorzugt 60 bis 80 Gew:-% einer Wasserphase,
b) bis 80 Gew.-%, vorzugsweise 10 bis 70 Gew.-%, besonders bevorzugt 20 bis 60 Gew.-%, insbesondere bevorzugt 30 bis 50 Gew.-% an Silikonöl,
c) 0,5 bis 20 Gew.-%, vorzugsweise 1 bis 15 Gew.-%, besonders bevorzugt 3 bis 10 Gew.-% eines oder mehrerer Emulgatoren und
d) 0,01 bis 5 Gew.-%, vorzugsweise 0,05 bis 3 Gew.-%, besonders bevorzugt 0,1 bis 2 Gew.-% an Copolymer A.

In einer weiteren besonders bevorzugten Ausführungsform liegen die erfindungsgemäßen Mittel als Wasser-in-Silikon-Emulsionen, vorzugsweise als kosmetische oder dermatologische Wasser-in-Silikon-Emulsionen, vor und enthalten bezogen auf das Gesamtgewicht der Mittel
a) bis zu 90 Gew.-%, vorzugsweise:20 bis 90 Gew.-%, besonders bevorzugt 40 bis 85 Gew.-%, insbesondere bevorzugt 60 bis 80 Gew.-% einer Wasserphase,
b) bis 80 Gew.-%, vorzugsweise 10 bis 70 Gew.-%, besonders bevorzugt 20 bis 60 Gew.-%, insbesondere bevorzugt 30 bis 50 Gew.-% an Silikonöl,
c) 0,5 bis 20 Gew.-%, vorzugsweise 1 bis 15 Gew.-%, besonders bevorzugt 3 bis 10 Gew.-% eines oder mehrerer Emulgatoren ausgewählt aus der Gruppe Cetyldimethiconecopolyol, Lauryldimethiconcopolyol, PEG/PPG-18/18 Dimethicon, Trimethylsilylamodimethicon und
d) 0,01 bis 5 Gew.-%, vorzugsweise 0,05 bis 3 Gew.-%, besonders bevorzugt 0,1 bis 2 Gew.-% an Copolymer A.

In einer weiteren bevorzugten Ausführungsform enthalten die erfindungsgemäßen Mittel eine oder mehrere UV-Filtersubstanzen.

In einer weiteren bevorzugten Ausführungsform enthalten die erfindungsgemäßen Mittel ein oder mehrere Hautbefeuchtungsmittel und/oder Farbstoffe und/oder farbgebende Pigmente.

Vorzugsweise werden die erfindungsgemäßen Mittel zur Pflege der Haut benutzt.

In einer weiteren bevorzugten Ausführungsform liegen die erfindungsgemäßen Mittel in Form von Suspensionen vor und enthalten bezogen auf das Gesamtgewicht der Mittel
a) 0,1 bis 10 Gew.-%, vorzugsweise 0,2 bis 6 Gew.-%, besonders bevorzugt 0,3 bis 5 Gew.-% an Copolymer A und
b) 0,1 bis 30 Gew.-%, vorzugsweise 0,5 bis 15 Gew.-%, besonders bevorzugt 1,0 bis 10 Gew.-% an festen Partikeln, insbesondere ausgewählt aus der Gruppe der Farbstoffe, farbgebenden Pigmente, Effekt- und Lichtschutzpigmente, Adsorbentien und Abrasivkomponenten.

In einer besonders bevorzugten Ausführungsform liegen die erfindungsgemäßen Mittel als Lidschatten auf Gelbasis vor und enthalten bezogen auf das Gesamtgewicht der Mittel
a) 0,1 bis 10 Gew.-%, vorzugsweise 0,2 bis 6 Gew.-%, besonders bevorzugt 0,3 bis 5 Gew.-% an Copolymer A und
b) 0,1 bis 30 Gew.-%, vorzugsweise 0,5 bis 15 Gew.-%, besonders bevorzugt 1,0 bis 10 Gew.-% an Farbstoffen und/oder farbgebenden Pigmenten.

Die erfindungsgemäßen Mittel können feste anorganische und organische Partikel enthalten. Für dekorative Kosmetika werden farbige und auch farblose Pigmente eingesetzt. Einige der nachfolgend genannten Pigmente dienen auch als UV-Absorber bzw. Lichtschutzpigmente.

Besonders bevorzugte Farbpigmente sind ausgewählt aus den Eisenoxiden mit den Colour-Index-Nummem Cl 77491 (Eisen rot), Cl 77492 (Eisenoxidhydrat gelb) und Cl 77499 (Eisenoxid schwarz), aus CI 77891 (Titandioxid) und Ruß. Andere bevorzugte Farbpigmente sind ausgewählt aus CI 15510, Cl 15585, CI 15850, Cl 15985, Cl 45170, Cl 45370, Cl 45380, Cl 45425, Cl 45430, CI 73360 und Cl 75470. Unter Effektpigmenten sind im Rahmen der vorliegenden Erfindung Pigmente zu verstehen, die durch ihre Brechungseigenschaften besondere' optische Effekte hervorrufen. Effektpigmente verleihen der behandelten Oberfläche (Haut, Haar, Schleimhaut) Glanz- oder Glittereffekte oder können durch diffuse Lichtstreuung Hautunebenheiten und Hautfältchen optisch kaschieren Als besondere Ausführungsform der Effektpigmente sind Interferenzpigmente bevorzugt.
Besonders geeignete Effektpigmente sind beispielsweise Glimmerpartikel, die mit mindestens einem Metalloxid beschichtet sind. Neben Glimmer, einem Schichtsilikat, sind auch Kieselgel und andere SiO₂-Modifikationen als Träger geeignet. Ein häufig zur Beschichtung verwendetes Metalloxid ist beispielsweise Titanoxid, dem gewünschtenfalls Eisenoxid beigemischt sein kann. Über die Größe und die Form (z.B. sphärisch, ellipsoid, abgeflacht, eben, uneben) der Pigmentpartikel sowie über die Dicke der Oxidbeschichtung können die Reflexionseigenschaften beeinflusst werden. Auch andere Metalloxide, z.B. Bismutoxychlorid (BiOCl), sowie die Oxide von beispielsweise Titan, insbesondere die TiO₂-Modifikationen Anatas und Rutil, sowie von Aluminium, Tantal, Niob, Zirkon und Hafnium. Auch mit Magnesiumfluorid (MgF₂) und Calciumfluorid (Flussspat, CaF₂) können Effektpigmente hergestellt werden.

Die Effekte lassen sich sowohl über die Partikelgröße als auch über die Partikelgrößenverteilung des Pigmentensembles steuern. Geeignete Partikelgrößenverteilungen reichen z.B. von 2 - 50 µm, 5 - 25 µm, 5 - 40 µm, 5 - 60 µm, 5 - 95 µm, 5-100 µm, 10-60µm, 10 - 100 µm, 10 - 125 µm, 20 - 100 µm, 20 - 150 µm, sowie < 15 µm. Eine breitere Teilchengrößenverteilung z.B. von 20 - 150 µm, ruft glitzernde Effekte hervor, während eine engere Teilchengrößenverteilung von < 15 µm für eine gleichmäßige seidige Erscheinung sorgt.

Die erfindungsgemäßen Mittel enthalten Effektpigmente vorzugsweise in Mengen von 0,1 ― 20 Gew.-%, besonders bevorzugt 0,5 ― 10 und insbesondere bevorzugt 1 ― 5 Gew.-%, jeweils bezogen auf das Gesamtgewicht des Mittels.

Bei den bevorzugten anorganischen Lichtschutzpigmenten handelt es sich um feindisperse oder kolloiddisperse Metalloxide und Metallsalze, beispielsweise Titanoxid, Zinkoxid, Eisenoxid, Aluminiumoxid, Ceroxid, Zirkoniumoxid, Silikate (Talk) und Bariumsulfat. Die Partikel sollten dabei einen mittleren Durchmesser von weniger als 100 nm, vorzugsweise zwischen 5 und 50 nm und besonders bevorzugt zwischen 15 und 30 nm äufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

Die bevorzugten anorganischen Partikelsubstanzen sind hydrophil oder amphiphil. Vorteilhafterweise können sie oberflächlich beschichtet, insbesondere oberflächlich wasserabweisend behandelt sein. Beispiele hiefür sind mit Aluminiumstearat beschichtete Titanoxid-Pigmente, mit Dimethylpolysiloxan (Dimethicone) beschichtetes Zinkoxid, mit Dimethicone beschichtetes Bornitrid und mit einem Gemisch aus Dimethylpolysiloxan und Silicagel und Aluminiumoxidhydrat beschichtetes Titanoxid, mit Octylsilanol beschichtetes Titanoxid oder sphärische Polyalkylsesquioxan-Partikel.

Bei organischen Lichtschutzpigmenten handelt es sich um bei Raumtemperatur kristallin vorliegende Substanzen, die in der Lage sind, ultraviolette Strahlen zu absorbieren und die aufgenommene Energie in Form längerwelligerer Strahlung, z.B. Wärme, wieder abzugeben. Man unterscheidet UVA-Filter und UVB-Filter. Die UVA- und UVB-Filter können sowohl einzein als auch in Mischungen eingesetzt werden. Die erfindungsgemäß geeigneten organischen UV-Filter sind ausgewählt aus den bei Raumtemperatur festen Derivaten von Dibenzoylmethan, Zimtsäureestern, Diphenylsäureestern, Benzophenon, Campher, p-Aminobenzoesäureestern, o-Aminobenzoesäureestern, Salicylsäureestern, Benzimidazolen, 1,3,5-Triazinen, monomeren und oligomeren 4,4-Diarylbutadiencarbonsäureestern und -carbonsäureamiden, Ketotricyclo(5.2.1.0)decan, Benzalmalonsäureestern sowie beliebigen Mischungen der genannten Komponenten. Die organischen UV-Filter können öllöslich oder wasserlöslich sein. Erfindungsgemäß besonders bevorzugte öllösliche UV-Filter sind 1-(4-tert-Butylphenyl)-3-(4'-methoxyphenyl)propan-1,3-dion, 1-Phenyl-3-(4'-Isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-octylhexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino(p-carbo-2'-ethyl-1'-hexyloxy)-1,3,5-triazin (Octyl Triazone) und Dioctyl Butamido Triazone sowie beliebige Mischungen der genannten Komponenten. Bevorzugte wasserlösliche UV-Filter sind 2-Phenylbenzimidazol-5-sulfonsäure und deren Alkali, Erdalkali, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z.B. 4-(2-oxo-3-bornylidenmethyl)benzolsulfonsäure und 2-Methyl-5-(2-oxo-3-bornyliden)sulfonsäure und deren Salze. In den erfindungsgemäßen Mitteln sind die anorganischen und organischen Lichtschutzpigmente in Mengen von 0,1 - 30 Gew.-%, vorzugsweise 1 - 20 Gew.-% und besonders bevorzugt 2 bis 15 Gew.-%, jeweils bezogen auf das Gesamtgewicht des Mittels, enthalten.

Die erfindungsgemäßen Mittel können teilchenförmige anorganische oder organische Adsorbentien mit mittleren Partikeldurchmessern von 1- 100 µm, enthalten. Die Adsorbentien sind ausgewählt aus pyrogenen Kieselsäuren, z.B. den Aerosol-Typen, Fällüngskieselsäuren, Kieselgelen, Siliciumdioxid, Toneh, z.B. Bentonite oder Kaolin, Magnesiumaiuminiumsiiikaten, z.B. Talkum und Bornitrid, gegebenenfalls modifizierte Stärken und Stärkederivate, Cellulosepulverh, Lactoglobulinderivaten, Polymerpulvern aus Polyolefinen, Polycarbonaten, Polyurethanen, Polyamiden, Polyestern, Polystyrolen, Polyacrylaten, (Meth)acrylat- oder (Meth)acrylat-Vinyliden-Copolymeren, die vernetzt sein können, Teflon oder Siliconen, sowie Mischungen der genannten Substanzen.

Die edihdungsgemäßen Mittel können Abrasivkomponenten, beispielsweise gemahlene Pflanzenteile wie Mandelkleie oder Weizenkleie, kristalliner Cellulose, gehärtetem Jojobaöl, Polymerkügelchen, bevorzugt aus Polyethylen oder Polyamid-11, mit mittleren Durchmessern von 90-600 µm, und aus wirkstoffhaltigen Mikro- oder Millikapsein, die petrochemische Polymere (z.B. aus Polyamid wie Nylon-11) und/oder Biopolymere wie Gelatine, Pektin, Pflanzlichen Gummen, Alginaten und Carrageenan enthalten. Bevorzugt eingesetzt werden Mandelkleie, Weizenkleie, gehärtetes Jojobaöl und Polyethylenkügelchen.

In einer weiteren bevorzugten Ausführungsform liegen die erfindungsgemäßen Mittel als Haarfärbe-, Haarbleich- und/oder -tönungsmittel vor und enthalten ein oder mehrere Substanzen ausgewählt aus Direktfarbstoffen, Oxidationsfarbstoffvorstufen und Bleichmitteln. In diesen Mitteln werden die Copolymere A in den üblichen pH-Bereichen, vorzugsweise als Verdicker und Stabilisator, eingesetzt.

An Direktfarbstoffen in Betracht kommen Nitroanilinderivate, wie 1-[(2-Hydroxyethyl)-amino]-2-nitrobenzol (Velsol® Yellow 2), 4-Hydroxypropylamino-3-Nitrophenol (Velsol® Red BN), 3-Nitro-p-Hydroxyethylaminophenol (Velsol® Red 54), 4-Hydroxyethylamino-3-Nitroanilin (Velsol® Red 3), N,N'-Bis-(Hydroxyethyl)-2-Nitro-p-Phenylendiamine (Velsol® Violet BS), N,N',N'-Tris-(Hydroxyethyl)-2-Nitro-p-Phenylendiamine (Velsol® Blue 2), 4-(2'-Hydroxyethyl)-amino-3-nitro-toluol, 4-(2'-Hydroxyethyl)-amino-3-nitro-benzylalkohol, 4-(2'-Hydroxyethyl)-amino-3-nitro-1-trifluormethyl-benzol, 4-(2',3'-Dihydroxypropyl)-amino-3-nitro-chlorbenzol, 4-(2'-Hydroxyethyl)-amino-3-nitro-brombenzol und 4-(2',3'-Dihydroxypropyl)-amino-3-nitro-brombenzol, Nitrobenzolderivate; beispielsweise 2-Amino-4-nitro-phenol, Pikräminsäure, 1-[(2'-Hydroxyethyl)-amino]-2-amino-4-nitrobenzol, 2-Nitro-4-[(2'-hydroxyethyl)amino]-anilin, 4-Bis-[(2'-hydroxyethyl)amino]-1-methylamino-2-nitrobenzol, 2,5-Bis-[(2'-hydroxyethyl)-amino]-nitrobenzol, 2-(2'-hydroxyethyl)-amino-4,6-dinitro-phenol; 1-Amino-4-(2',3'-dihydroxypropyl)-amino-2-nitro-5-chlor-benzol, aber auch Triphenylmethanfarbstoffe, wie zum Beispiel Basic Violet 1 (C.I. 42535), Azofarbstoffe, wie zum Beispiel Acid Brown 4 (C.l. 14805), Anthrachinonfarbstoffe, wie zum, Beispiel Disperse Blue 23 (C.I.61545), Disperse Violet 4 (C.I.61105)-1,4,5,8-Tetraaminoanthrachinon und 1,4-Diaminoanthrachinon und weitere direktziehende Farbstoffe;

An Oxidationsfarbstoffvorstufen stehen zur Verfügung p-Phenylendiamine und p-Aminophenole oder deren Derivate wie beispielsweise p-Toluylendiamin, p-Phenylendiamin, p-Aminophenol, die zum Zweck der Nuancierung der Färbung mit sogenannten Modifiern oder Kupplern, wie zum Beispiel m-Phenylendiamin, Resorcin, m-Aminophenol und deren Derivaten kombiniert werden.

Als Oxidationsmittel zur Entwicklung der Haarfärbungen oder als Bleichmittel kommen vorzugsweise Hydrogenperoxyd und dessen Additionsverbindungen in Betracht. In einer weiteren bevorzugten Ausführungsform liegt das erfindungsgemäße Mittel als Haarbleichmittel vor und enthält ein oder mehrere Oxidationsmittel, vorzugsweise Wasserstoffperoxid.

Besonders vorteilhaft ist die stabilisierende Wirkung der Copolymere A auf die Oxidationsmittel.

Zur Erhöhung der Farbintensität können die erfindungsgemäßen Mittel die in kosmetischen Systemen üblichen Carrier enthalten, insbesondere Benzylalkohol, Vanillin (4-Hydroxy-3-methoxy-benzaldehyd), Isovanillin, p-Hydroxyanisol, 3-Hydroxy-4-methoxy-benzaldehyd, 2-Phenoxyethanol, Salicylaldehyd, 3,5-Dihydroxybenzaldehyd, 3,4-Dihydroxybenzaldehyd, 4-Hydroxyphenylacetamid, p-Hydroxybenzoe-säuremethylester, p-Hydroxybenzaldehyd, m-Kresol, Hydrochinonmonomethylether, o-Fluorphenol, m-Fluorphenol, p-Fluorphenol, 2-(2-Hydroxyphenoxy)-ethanol, 3,4-Methylendioxyphenol, Resorcinmonomethylether, 3,4-Dimethoxyphenol, 3-Trifluormethylphenol, Resorcinmonoacetat, Ethylvanillin, 2-Thiophenethanol, Milchsäurebutylester und Glycolsäurebutylester.

Die erfindungsgemäßen Haarfärbemittel können vorteilhafterweise perlglanzgebende Verbindungen enthalten, beispielsweise Fettsäuremonoalkanolamide, Fettsäuredialkanolamide, Monoester oder Diester von Alkylenglycol, insbesondere Ethylenglykol und/oder Propylenglykol oder dessen Oligomere mit höheren Fettsäuren, z.B. Palmitinsäure, Stearinsäure oder Behensäure oder Mischungen davon, Mono- oder Diester von Alkylenglykolen mit Fettsäuren, Fettsäuren und deren Metallsalze, Monoester oder Polyester von Glycerin mit Carbonsäuren und Ketosulfone verschiedener Art, bevorzugt Ethylenglycoldistearat und Polyethylenglycoldistearat mit ca. 3 Glykoleinheiten.

In einer weiteren bevorzugten Ausführungsform liegen die erfindungsgemäßen Mittel als Deodorantien oder Antiperspirantien vor. Vorzugsweise werden die Copolymere A hierbei als Verdicker, Konsistenzgeber, Emulgator, Solubilisator, Dispergator, Gleitmittel, Haftmittel, Conditioner und Stabilisator eingesetzt.

Die erfindungsgemäßen Deodorantien und Antiperspirantien enthalten, bezogen auf die fertigen Mittel, bevorzugt 0,01 bis 10 Gew.-%, besonders bevorzugt 0,1 bis 5 Gew.-%, insbesondere bevorzugt 0,5 bis 3 Gew.-% der Copolymere A.

In einer weiteren bevorzugten Ausführungsform enthalten die erfindungsgemäßen Mittel 0,01 bis 89 Gew.-%, besonders bevorzugt 5 bis 50 Gew.-%, insbesondere bevorzugt 10 bis 30 Gew.-% Wasser.

In einer weiteren bevorzugten Ausführungsform enthalten die erfindungsgemäßen Mittel bis 10 Gew.-%, bevorzugt 1 bis 6 Gew.-% Glycerin.

In einer weiteren bevorzugten Ausführungsform enthalten die erfindungsgemäßen Mittel antimikrobielle Wirkstoffe, die die schweißzersetzenden Mikroorganismen bzw. das schweißzersetzende Esterase-Enzym hemmen.
Bevorzugt geeignet als antimikrobielle Wirkstoffe sind Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol und Kombinationen dieser Wirksubstanzen.
Die erfindungsgemäßen Mittel enthalten die antimikrobiellen Mittel bevorzugt in Mengen bis 50 Gew.-%, besonders bevorzugt 0,01 bis 10 Gew.-%, insbesondere bevorzugt 0,1 bis 10 Gew.-%.

In einer weiteren bevorzugten Ausführungsform enthalten die erfindungsgemäßen Mittel Adstringentien.
Bevorzugte Adstringentien sind Oxide, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzug von Calcium, Magnesium, Aluminium, Titan, Zirkon oder Zink.

Die erfindungsgemäßen Mittel enthalten die adstringenden Wirkstoffe bevorzugt in Mengen 0 bis 50 Gew.-%, besonders bevorzugt 0,01 bis 10 Gew.-% und insbesondere bevorzugt 0,1 bis 10 Gew.-%.

In einer weiteren bevorzugten Ausführungsform enthalten die erfindungsgemäßen Mittel Gelierungsmittel.

Als Gelierungsmittel eignen sich alle oberflächenaktiven Stoffe, die in der flüssigen Phase gelöst eine Netzwerkstruktur ausbilden und so die flüssige Phase verfestigen. Geeignete Geliermittel sind z.B. in WO 98/58625 genannt.
Bevorzugte Gelierungsmittel sind Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, - lauryl-, 16-Hydroxyhexadecanoylsäure; Fettsäureamide; Fettsäurealkanolamide; Dibenzalsorbit und alkohollösliche Polyamide und Polyacrylamide oder Mischungen solcher.
Bevorzugt enthalten die erfindungsgemäßen Mittel 0,01 bis 20 Gew.-%, besonders bevorzugt 0,1 bis 10 Gew.-%, insbesondere bevorzugt 1 bis 8 Gew.-% und ganz besonders bevorzugt 3 bis 7 Gew.-% an Geliermitteln.

Die Copolymere A sind hervorragende Verdicker für wässrige, wässrig-alkoholische oder wässrig-tensidische Formulierungen und verleihen den Mitteln ein im Vergleich zu Aristoflex® AVC transparenteres Erscheinungsbild.

Die Einsatzmenge der Copolymere A in Mitteln auf rein wässriger, wässrig-alkoholischer oder wässrig-tensidischer Basis liegt vorzugsweise im Bereich von 0,01 bis 10 Gew.-%, besonders bevorzugt 0,1 bis 5 Gew.-%, insbesondere bevorzugt 0,5 bis 3 Gew.-%.

Die Copolymere A haben vielfältige Einsatzmöglichkeiten und eignen sich beispielsweise für den Einsatz in wässrigen, wässrig-alkoholischen, wässrigtensidischen Formulierungen, Emulsionen, Suspensionen, Dispersionen und Pudern.

In einer bevorzugten Ausführungsform werden die Copolymere A in Rinse-off Produkten, bevorzugt Shampoos, Duschbäder, Duschgels und Schaumbäder, eingesetzt.

In einer weiteren bevorzugten Ausführungsform werden die Copolymere A in Leaveon Produkten, bevorzugt Hautpflegemittel Tagescremes, Nachtcremes, Pflegecremes, Nährcremes, Bodylotions, Salben, Sonnenschutzmittel, Lippenpflegemittel und Deodorantien, eingesetzt.

In einer weiteren bevorzugten Ausführungsform liegen die erfindungsgemäßen Mittel als tensidfreie, wässrige Mittel und Emulsionen, beispielsweise als Haarpflegemittel, Haarkuren und Haarspülungen, Haargele, Haarstylingmittel aber auch als Dauerwellenmittel, Haarfärbemittel, sowie als dekorative Kosmetika, beispielsweise make-ups, eye-shadows, Lippenstifte, Mascara und dergleichen vor. In einer besonders bevorzugten Ausführungsform liegen die erfindungsgemäßen Mittel als Haarfärbe-, -bleich-, -pflege- oder -stylingmittel vor.

Die erfindungsgemäß eingesetzten Copolymere A eignen sich zur Herstellung von pumpbaren, sprühbaren und/oder verschäumbaren Non-Aerosol Produkten. In einer bevorzugten Ausführungsform liegen die erfindungsgemäßen Mittel daher in einer pumpbaren, sprühbaren und/oder verschäumbaren Form vor. Vorzugsweise enthalten diese Mittel kein Aerosol.

In einer besonders bevorzugten Ausführungsform werden die Copolymere A in versprühbare, pumpbare und verschäumbare Gele und Schäume, insbesondere in versprühbare Haargele und verschäumbare Sonnenschutzmittel eingearbeitet und bewirken eine Verbesserung des Sprühverhaltens der Mittel mit optimierter Tröpfchengrößenverteilung.

Vorteilhaft ist eine Haargelzusammensetzung, enthaltend mindestens ein Copolymer A und mindestens ein haarfestigendes Polymer.

Die Viskosität der Gele beträgt vorzugsweise 100 bis 5000 mPa*s, besonders bevorzugt 200 bis 1000 mPa*s, insbesondere bevorzugt 250 bis 800 mPas*s, gemessen als dynamische Viskositätsmessung mit einem Bohlin Rheometer CS, Messkörper C25 bei einer Temperatur von 25°C und einer Schergeschwindigkeit von 50 s⁻¹.

Das gelbildende Copolymer A wird vorzugsweise in einer Menge von 0,1 bis 10, besonders bevorzugt von 0,2 bis 8 Gew.-% und das haarfestigende Polymer in einer Menge von vorzugsweise 0,1 bis 15, besonders bevorzugt von 0,5 bis 10 Gew.-% eingesetzt.

Das haarfestigende Polymer kann nichtionisch, anionisch, kationisch oder amphoter sein, ist aber vorzugsweise nichtionisch oder anionisch. Es kann ein synthetisches oder ein natürliches Polymer sein. Unter natürlichen Polymeren werden auch chemisch modifizierte Polymere natürlichen Ursprungs verstanden. Bevorzugt sind insbesondere solche Polymere, die eine ausreichende Löslichkeit in Wasser, Alkohol oder Wasser/Alkohol-Gemischen besitzen, um in dem erfindungsgemäßen Mittel in vollständig gelöster Form vorzuliegen. Unter haarfestigenden Polymeren werden solche Polymere verstanden, die bei Anwendung in 0,01 bis 5 %iger wässriger, alkoholischer oder wässrig-alkoholischer Lösung oder Dispersion in der Lage sind, auf dem Haar eine haarfestigende Wirkung zu erzielen.
Geeignete synthetische, nichtionische haarfestigende Polymere sind Homo- oder Copolymere, die aus mindestens einem der folgenden Monomere aufgebaut sind: Vinylpyrrolidon, Vinylimidazol, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Dialkylaminoalkylmethacrylamid, Dialkylaminoalkylacrylamid, Alkylacrylat, Alkylmethacrylat, Propylenglycol oder Ethylenglycol, wobei die Alkylgruppen dieser Monomere vorzugsweise C₁- bis C₇-Alkylgruppen, besonders bevorzugt C₁- bis C₃-Alkylgruppen sind. Geeignet sind z.B. Homopolymere des Vinylcaprolactams, des Vinylpyrrolidons oder des N-Vinylformamids. Weitere geeignete haarfestigende Polymere sind z.B. Copolymerisate aus Vinylpyrrolidon und Vinylacetat, Terpolymere aus Vinylpyrrolidon, Vinylacetat und Vinylpropionat, Terpolymere aus Vinylpyrrolidon, Vinylcaprolactam und Dialkylaminoalkyl-(meth)acrylat, Terpolymere aus Vinylpyrrolidon, Vinylcaprolactam und Dialkylaminoalkyl(meth)acrylamid, Terpolymere aus Vinylpyrrolidon, Vinylimidazol und (Meth)acrylamid, Polyacrylamid, Polyvinylalkohol, sowie haarfestigende Polyethylenglycol/Polypropylenglykol Copolymere. Besonders bevorzugte nichtionische Polymere sind Polyvinylpyrrolidon und Polyvinylpyrrolidon/Vinylacetat Copolymere. Bevorzugt sind nichtionische Vinyllactam Homo- und Copolymere. Geeignete Vinyllactame sind z.B. Vinylcaprolactam und Vinylpyrrolidon. Besonders bevorzugt sind Polyvinylpyrrolidon, Polyvinylcaprolactam, Terpolymere aus Vinylpyrrolidon, Vinylimidazol und (Meth)acrylamid und Vinylpyrrolidon/Vinylacetat Copolymere. Bevorzugte Handelsprodukte sind Luviskol® VA 37 Luviskol® VA 64 und Luviset® Clear.
Geeignete anionische haarfestigende Polymere können natürliche oder synthetische Homo- oder Copolymere mit Säuregruppen enthaltenden Monomereinheiten sein, welche gegebenenfalls mit Comonomeren, die keine Säuregruppen enthalten, copolymerisiert sind. Die Säuregruppen sind vorzugsweise ausgewählt aus -COOH, -SO₃H, -OSO₃H, -OPO₂H und -OPO₃H₂, von denen die Carbonsäuregruppen bevorzugt sind. Die Säuregruppen können unneutralisiert, teilweise oder vollständig neutralisiert vorliegen. Sie liegen vorzugsweise zu 50 bis 100 % in anionischer bzw. neutralisierter Form vor. Als Neutralisationsmittel können die oben genannten Neutralisationsmittel verwendet werden. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind beispielsweise Acrylsäure, Methacrylsäure, Crotonsäure, Maleinsäure bzw. Maleinsäureanhydrid oder deren Monoester, Aldehydocarbonsäuren oder Ketocarbonsäuren.
Nicht mit Sulfogruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglycol oder Ethylenglycol, aminsubstituierte Vinylmonomere wie z.B. Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C₁- bis C₇-Alkylgruppen, besonders bevorzugt C₁- bis C₃-Alkylgruppen sind.
Geeignete anionische Polymere sind insbesondere Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure, sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.
Bevorzugte anionische Polymere sind vernetzte oder unvernetzte Vinylacetat/Crotonsäure Copolymere. Ebenso bevorzugt sind partial veresterte Copolymere zwischen Vinylmethylether und Maleinsäureanhydrid. Weitere geeignete anionische Polymere sind z.B. Terpolymere aus Acrylsäure, Alkylacrylat und N-Alkylacrylamid, insbesondere Acrylsäure/Ethylacrylat/N-t-Butylacrylamid Terpolymere oder Terpolymere aus Vinylacetat, Crotonat und Vinylalkanoat, insbesondere Vinylacetat/Crotonat/Vinylneodecanoat Copolymere.
Geeignete filmbildende amphotere Polymere sind Polymere, welche neben sauren oder anionischen Gruppen als weitere funktionelle Gruppen basische oder kationische Gruppen, insbesondere primäre, sekundäre, tertiäre oder quaternäre Amingruppen enthalten. Beispiele hierfür sind Copolymere gebildet aus Alkylacrylamid, Alkylaminoalkylmethacrylat und zwei oder mehr Monomeren ausgewählt aus Acrylsäure, Methacrylsäure oder deren Ester, wobei die Alkylgruppen 1 bis 4 C-Atome enthalten, mindestens eines der Monomere eine Säuregruppe aufweist.
Weitere Beispiele für geeignete haarfestigende Polymere sind Copolymere von Acrylsäure, Methacrylat und Methacrylamidopropyltrimethylammoniumchlorid, Copolymere aus Acrylamidopropyltrimethylammoniumchlorid und Acrylaten, Copolymere aus Acrylamid, Acrylamidopropyltrimethylammoniumchlorid, 2-Amidopropylacrylamidsulfonat und Dimethylaminopropylamin oder Chitosane. Geeignet sind auch Polymere mit Betaingruppen tragenden Monomeren wie z.B. Copolymere aus Methacryloylethylbetain und zwei oder mehr Monomeren von Acrylsäure oder deren einfachen Estern, bekannt unter der INCI-Bezeichnung Methacryloyl Ethyl Betain/ Acrylat Copolymer.
In einer bevorzugten Ausführungsform wird das erfindungsgemäße Mittel in einem wässrigen, einem alkoholischen oder in einem wässrig-alkoholischen Medium mit vorzugsweise mindestens 10 Gew.-%, besonders bevorzugt mindestens 50 Gew.-% Wasser und vorzugsweise maximal 40 Gew.-% Alkohol konfektioniert.

Als Alkohole können insbesondere die für kosmetische Zwecke üblicherweise verwendeten niederen Monoalkohole mit 1 bis 4 C-Atomen wie z.B. Ethanol und Isopropanol enthalten sein.
Die erfindungsgemäßen Haargele sind klare, transparente oder durchscheinende, farblose Mittel mit besonders positiven Sprüheigenschaften.

Die erfindungsgemäßen kosmetischen und pharmazeutischen Mittel können anionische, kationische, nichtionische, zwitter-ionische und/oder amphotere Tenside enthalten.

Die Gesamtmenge der in den erfindungsgemäßen Mitteln (z.B. im Falle von Rinse-Off-Produkten) eingesetzten Tenside beträgt, bezogen auf das Gesamtgewicht der Mittel, vorzugsweise 2 bis 70 Gew.-%, besonders bevorzugt 5 bis 40 Gew.-%, insbesondere bevorzugt 12 bis 35 Gew.-%.

Als anionische Tenside bevorzugt sind (C₁₀-C₂₀)-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester,
Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Der Gewichtsanteil der anionischen Tenside beträgt, bezogen auf das Gesamtgewicht der Mittel, vorzugsweise 2 bis 30 Gew.-%, besonders bevorzugt 5 bis 25 Gew.-%, insbesondere bevorzugt 12 bis 22 Gew.-%.

Bevorzugte kationische Tenside sind quartäre Ammonium-Salze, wie Di-(C₁₀-C₂₄)-Alkyl-dimethylammoniumchlorid oder -bromid, vorzugsweise Di-(C₁₂-C₁₈)-Alkyl-dimethylammoniumchlorid oder -bromid; (C₁₀-C₂₄)-Alkyl-dimethylethylammoniumchlorid oder-bromid; (C₁₀-C₂₄)-Alkyl-trimethylammoniumchlorid oder -bromid, vorzugsweise Cetyltrimethylammoniumchlorid oder-bromid und (C₂₀-C₂₂)-Alkyl-trimethylammoniumchlorid oder -bromid; (C₁₀-C₂₄)-Alkyl-dimethylbenzylammoniumchlorid oder -bromid, vorzugsweise (C₁₂-C₁₈)-Alkyl-dimethylbenzylammoniumchlorid; N-(C₁₀-C₁₈)-Alkyl-pyridiniumchlorid oder-bromid, vorzugsweise N-(C₁₂-C₁₆)-Alkyl-pyridiniumchlorid oder -bromid; N-(C₁₀-C₁₈)-Alkyl-isochinoliniumchlorid, -bromid oder -monoalkylsulfat; N-(C₁₂-C₁₈)-Alkyl-polyoylaminoformylmethylpyridiniumchlorid; N-(C₁₂-C₁₈)-Alkyl-N-methyl-morpholinium-chlorid, -bromid oder -monoalkylsulfat; N-(C₁₂-C₁₈)-Alkyl-N-ethyl-morpholinium-chlorid, -bromid oder -monoalkylsulfat; (C₁₆-C₁₈)-Alkyl-pentaoxethyl-ammonium-chlorid; Diisobutylphenoxyethoxyethyldimethylbenzylammonium-chlorid; Salze des N,N-Diethylaminoethylstearylamids und -oleylamids mit Salzsäure, Essigsäure, Milchsäure, Zitronensäure, Phosphorsäure; N-Acyl-aminoethyl-N,N-diethyl-N-methylammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Der Gewichtsanteil der kationischen Tenside beträgt, bezogen auf das Gesamtgewicht der Mittel, vorzugsweise 1 bis 10 Gew.-%, besonders bevorzugt 2 bis 7 Gew.-%, insbesondere besonders bevorzugt 3 bis 5 Gew.-%.

Als nichtionische Tenside bevorzugt sind Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics® ); Fettsäurealkylolamide, (Fettsäureamidpolyethylenglykole); N-Alkyl-, N-Alkoxypolyhydroxyfettsäureamid, Saccharoseester; Sorbitester und der Polyglykolether.

Der Gewichtsanteil der nichtionischen Tenside in den erfindungsgemäßen Mitteln (z.B. im Falle von Rinse-off-Produkten) liegt, bezogen auf das Gesamtgewicht der Mittel, vorzugsweise im Bereich von 1 bis 20 Gew.-%, besonders bevorzugt von 2 bis 10 % und insbesondere bevorzugt von 3 bis 7 Gew.-%.

Bevorzugte Amphotenside sind N-(C₁₂-C₁₈)-Alkyl-β-aminopropionate und N-(C₁₂₋C₁₈)-Alkyl-β-iminodipropionate als Alkali- und Mono-, Di- und Trialkylammonium-Salze; N-Acylaminoalkyl-N,N-dimethyl-acetobetain, vorzugsweise N-(C₈-C₁₈)-Acyl-aminopropyl-N,N-dimethylacetobetain; (C₁₂-C₁₈)-Alkyl-dimethyl-sulfopropyl-betain; Amphotenside auf Basis Imidazolin (Handelsname: Miranol® , Steinapon® ), vorzugsweise das Natrium-Salz des 1-(β-Carboxy-methyloxyethyl)-1-(carboxymethyl)-2-lauryl-imidazoliniums; Aminoxid, z.B. (C₁₂-C₁₈)-Alkyldimethylaminoxid, Fettsäureamidoalkyl-dimethylaminoxid.

Der Gewichtsanteil der amphoteren Tenside beträgt, bezogen auf das Gesamtgewicht der Mittel, vorzugsweise 0,5 bis 20 Gew.-% und besonders bevorzugt 1 bis 10 Gew.-%.

Bevorzugte Tenside sind Laurylsulfat, Laurethsulfat, Cocoamidopropylbetain, Natriumcocoylglutamat, Lauroamphoacetat.

In einer bevorzugten Ausführungsform enthalten die Mittel zusätzlich noch als schaumverstärkende Mittel Co-Tenside aus der Gruppe der Alkylbetaine, Alkylamidobetaine, Aminopropionate, Aminoglycinate, lmidazoliniumbetaine und Sulfobetaine, Aminoxide, Fettsäurealkanolamide und Polyhydroxyamide.

Die erfindungsgemäßen Mittel können als weitere Hilfs- und Zusatzstoffe in der Kosmetik gebräuchliche Zusätze, wie z.B. kationische Polymere, Filmbildner, Überfettungsmittel, Stabilisatoren, biogene Wirkstoffe, Glycerin, Konservierungsmittel, Perlglanzmittel, Farb- und Duftstoffe, Lösungsmittel, Trübungsmittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des Weiteren können den erfindungsgemäßen Mitteln antimikrobiell wirkende Agentien zugesetzt werden.

Als kationische Polymere eignen sich die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVPdimethylaminoethylmethacrylat-Copolymer, Guar-hydroxypropyltriammoniumchloride, sowie Calciumalginat und Ammoniumalginat. Des weiteren können eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan. Geeignete Silikonverbindungen sind beispielsweise Dimethylpolysiloxan, Methylphenylpolysiloxane, cyclische Silicone und amino-, fettsäure-, alkoholpolyether-, epoyx-, fluor- und/oder alkylmodifizierte Siliconverbindungen, sowie Polyalkylsiloxane, Polyalkylarylsiloxane, Polyethersiloxan-Copolymere, wie in US 5,104,645 und den darin zitierten Schriften beschrieben, die bei Raumtemperatur sowohl flüssig als auch harzförmig vorliegen können. Geeignete Filmbildner sind, je nach Anwendungszweck wasserlösliche

Polyurethane, beispielsweise C₁₀-Polycarbamylpolyglycerylester, Polyvinylalkohol, Polyvinylpyrrolidon, -copolymere, beispielsweise Vinylpyrrolidon/Vinylacetatcopolymer, wasserlösliche Acrylsäurepolymere/Copolymere bzw. deren Ester oder Salze, beispielsweise Partialestercopolymere der Acryl/ Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carboxyvinyl-Polymere, wie Carbomere und deren Salze, Polysaccharide, beispielsweise Polydextrose und Glucan.

Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Als feuchtigkeitsspendende Substanz stehen beispielsweise Isopropylpalmitat, Glycerin und/oder Sorbitol zu Verfügung.

Als Stabilisatoren können Metallsalze von Fettsäuren, wie z.B. Magnesium-, Aluminium- und/oder Zinkstearat eingesetzt werden.

Unter biogenen Wirkstoffen sind beispielsweise Pflanzenextrakte und Vitaminkomplexe zu verstehen.

Zusätzlich können die erfindungsgemäßen Mittel organische Lösungsmittel enthalten. Prinzipiett kommen als organische Lösungsmittel alle ein-oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin (Glycerintriacetat) und 1-Methoxy-2-propanol.

Die erfindungsgemäßen Mittel können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen als Pflegezusatz abgemischt werden.

Als UV-Filter eignen sich z.B. 4-Aminobenzoesäure; 3-(4'-Trimethylammonium)benzyliden-boran-2-on-methylsulfat; 3,3,5-Trimethyl-cyclohexylsalicylat; 2-Hydroxy-4-methoxybenzophenon; 2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4'-Sulfo)-benzyliden-bornan-2-on und seine Salze; 2-Cyan-3,3-diphenyl-acrylsäure-(2-ethylhexylester); Polymer von N-[2(und 4)-(2-oxoborn-3-ylidenmethyl)benzyl]-acrylamid; 4-Methoxy-zimtsäure-2-ethyl-hexylester; ethoxyliertes Ethyl-4-amino-benzoat; 4-Methoxy-zimtsäure-isoamylester; 2,4,6-Tris-[p-(2-ethylhexyloxycarbonyl)anilino]-1,3,5-triazin; 2-(2H-benzotriazol-2-yl)-4-methyl-6-(2-methyl-3-(1,3,3,3-tetramethyl-1-(trimethylsilyloxy)-disiloxanyl)propyl)phenol; 4,4'-[(6-[4-((1,1-dimethylethyl)-aminocarbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz; und/oder 4-Isopropylbenzylsalicylat.

Vorteilhafte erfindungsgemäße Mittel enthalten ein oder mehrere Antioxidantien.

Als günstige, aber dennoch fakultativ zu verwendende Aritioxidantien können alle für kosmetische, dermatologische und/oder pharmazeutische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, Y-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid), Superoxid-Dismutase und die erfindungsgemäß geeigneten Derivate (Salze, Ester, Ether, Zucker, Nukleotide, Nukleoside, Peptide und Lipide) dieser genannten Stoffe.

Besonders vorteilhaft im Sinne der vorliegenden Erfindung können wasserlösliche Antioxidantien eingesetzt werden.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge der Antioxidantien (eine oder mehrere Verbindungen) in den erfindungsgemäßen Mitteln beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Mittel. Sofern Vitamin E und/oder dessen Derivate das oder die Antioxidantien darstellen, ist es vorteilhaft, deren jeweilige Konzentrationen aus dem Bereich von 0,001 bis 10 Gew.-%, bezogen auf das Gesamtgewicht der Mittel zu wählen.

Sofern Vitamin A, bzw. Vitamin-A-Derivate, bzw. Carotine bzw. deren Derivate das oder die Antioxidantien darstellen, ist es vorteilhaft, deren jeweilige Konzentrationen aus dem Bereich von 0,001 bis 10 Gew.-%, bezogen auf das Gesamtgewicht der Mittel, zu wählen.

In einer besonders bevorzugten Ausführungsform der Erfindung enthalten die kosmetischen oder pharmazeutischen Mittel Antioxidantien ausgewählt aus Superoxid-Dismutase, Tocopherol (Vitamin E) und Ascorbinsäure (Vitamin C).

Als Konservierungsmittel in Betracht kommen beispielsweise Phenoxyethanol, Parabene, Pentandiol oder Sorbinsäure.

Als Farbstoffe können die für kosmetische Zwecke geeigneten und zugelassenen Substanzen verwendet werden. Als antifugide wirkstoffe eignen sich bevorzugt ketoconazol, Oxiconazol, Terbinafin, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Zn-Pyrithion und Octopirox® .

Um die rheologischen Eigenschaften von wässrigen oder lösungsmittelhaltigen Emulsionen oder Suspensionen einzustellen, werden in der Fachliteratur eine Vielzahl von unterschiedlichen Systemen angegeben. Bekannt sind beispielsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrine. Als synthetische Polymere kommen verschiedene Materialien zum Einsatz, wie z.B. Polyvinylalkohole, Polyacrylamide, Polyvinylamide, Polysulfonsäuren, Polyacrylsäure, Polyacrylsäureester, Polyvinylpyrrolidon, Polyvinylmethylether, Polyethylenoxide, Copolymere aus Maleinsäureanhydrid und Vinylmethylether, sowie diverse Mischungen und Copolymerisate aus den o.a. Verbindungen, einschließlich ihrer verschiedenen Salze und Ester. Diese Polymere können wahlweise vernetzt oder unvernetzt sein.

Wesentlich für die Erfindung ist, dass die Copolymere A auch ohne Mitverwendung eines zusätzlichen Co-Emulgators und/oder ohne Mitverwendung eines zusätzlichen Konsistenzgebers eingesetzt werden können. Die Mitverwendung von Co-Emulgatoren und/oder Konsistenzgebern ist daher nicht zwingend, aber möglich.
Eine Kombination mit anderen bekannten Co-Emulgatoren und/oder Konsistenzgebern kann zur Einstellung spezieller kosmetischer Profile und zur Ausnutzung synergistischer Effekte wünschenswert sein.

Die erfindungsgemäßen Mittel sind üblicherweise auf einen pH Wert im Bereich 2 bis 12, bevorzugt pH 3 bis 8, eingestellt.

Die Beschaffenheit der erfindungsgemäßen Mittel ist ausgesprochen vorteilhaft: die Emulsionen sind cremig und salbig und haben überhaupt nicht das gelartige oder sogar gelatineartige Aussehen gewisser Emulsionen nach dem Stand der Technik, bei denen die äußere wässrige Phase verdickt ist.

Auch das kosmetische Gefühl auf der Haut ist sehr gut. Beim Auftragen verleiht die Emulsion ein Gefühl der Frische und des Komforts, wobei sie gleichzeitig gehaltvoll und nährend wirkt; sie ist weich und komfortabel und in keiner Weise klebrig.

Die erfindungsgemäßen Mittel können aerosolfrei in gut sprühbarer Form konfektioniert werden.

Die nachfolgenden Beispiele und Anwendungen sollen die Erfindung näher erläutern, ohne sie jedoch darauf zu beschränken (bei allen Prozentangaben handelt es sich um Gewichts-%).

### Beispiele

Herstellung der Copolymere

### Beispiel A

In einem 1000 ml Kolben mit Ankerrührer, Rückflußkühler, Innenthermometer, Einleitungsmöglichkeit für N₂ und NH₃ wurden 501 g tert.-Butanol und 14 g Wasser vorgelegt. Anschließend wurden 80,00 g 2-Acrylamido-2-methyl-propan-sulfonsäure eingetragen und unter starkem Rühren dispergiert, wobei eine Trübung des Lösungsmittels erhalten blieb. Über einen Zeitraum von 30 Minuten leitete man 6,64 g Ammoniak in den überstehenden Gasraum ein und rührte mindestens weitere 30 Minuten nach bis sich ein pH-Wert von 6 bis 7 eingestellt hatte. Man gab 4,10 g N-Vinylcaprolactam und 0,8 g Methacrylsäureallylester hinzu und spülte die Vorlage jeweils mit tert.-Butanol (ca. 6 ml) nach, um Verluste bei der Zugabe zu minimieren. Das Reaktionsgemisch wurde dann auf eine Temperatur von 60°C erwärmt, wobei die Reaktionsmischung durch gleichzeitiges Einleiten von N₂ inertisiert wurde. Nach Erreichen der Temperatur von 60°C wurden 1,0 g Dilauroylperoxid zugegeben. Die Reaktion sprang unmittelbar nach Zugabe des Initiators an, was an einem Anstieg der Temperatur und am Ausflocken des Polymers zu erkennen war. Etwa 15 Minuten nach dem Einsetzen der Polymerisationsreaktion wurde die Stickstoffzufuhr abgestellt. Ungefähr 30 Minuten nach Zugabe des Starters Dilauroylperoxid erreichte die Temperatur ein Maximum (ca. 65 - 70°C). Weitere 30 Minuten nach Durchlaufen dieses Maximums wurde zum Rückfluß erhitzt und unter diesen Bedingungen zwei Stunden nachgerührt. Der Inhalt des Reaktionsgefäßes nahm im Verlauf der Reaktion eine breiartige Konsistenz an, war aber noch gut rührbar. Anschließend wurde auf Raumtemperatur abgekühlt und der Feststoff abgesaugt. Die Paste wurde bei 60 - 70°C über 24 Stunden im Vakuumtrockenschrank getrocknet. Man erhielt 92,2 g eines feinen weißen Pulvers.

### Beispiel B

Entsprechend Beispiel A wurde das vernetzte Copolymer aus 55 g 2-Acrylamido-2-methyl-propan-sulfonsäure, 35 g N-Vinylcaprolactam und 1,9 g Trimethylolpropantrimethacrylat hergestellt.

### Beispiel C

Entsprechend Beispiel A wurde das vernetzte Copolymer aus 80 g 2-Acrylamido-2-methyl-propan-sulfonsäure, 4,2 g N-Vinylcaprolactam und 1,8 g Trimethylolpropantriacrylat hergestellt.

### Hautpflegemittel

In den folgenden Anwendungsbespielen 1 bis 25 wurden jeweils die Copolymere aus den Beispielen A, B und C verwendet. Diese Beispiele werden entsprechend mit Beispiel 1A (enthaltend das Copolymer aus Beispiel A), 1B (enthaltend das Copolymer aus Beispiel B), 1C (enthaltend, das Copolymer aus Beispiel C), 2A (enthaltend das Copolymer aus Beispiel,A) usw. bezeicünet.

Beispiele 1A; 1B und 1C: Hautmilch, sprühbar

| | | | |
|---|---|---|---|
| A | Hostaphat® KL 340 D | (Clariant) | 1.00 % |
| | Trilaureth-4 Phosphat | | |
| | Mineralöl, niedrigviskos | | 8.00 % |
| | Isopropyl Palmitat | | 3.00 % |
| | Cetearyl Alcohol | | 0.50 % |
| | Myritol® 318 | | 2.00 % |
| | Caprylic/Capric Triglyceride | | |
| | Tegin® M | | 0.50 % |
| | Glyceryl Stearat | | |
| | SilCare® Silicone 41 M15 | (Clariant) | 1.00 % |
| | Caprylyl Methicone | | |
| B | Copolymer aus Beispiel A, B oder C | | 0.40 % |
| C | Wasser | | ad 100.00 % |
| | Glycerin | | 5.00 % |
| D | Duftstoff | | 0.30 % |
| | Alkohol | | 5.00 % |
| | Tocopheryl Acetat | | 1.00 % |
| E | Nipaguard® PDU | (Clariant) | q.s. |
| | Propylene Glycol (und) Diazolidinyl Harnstoff (und) Methylparaben (und) Propylparaben | | |

### Herstellung:

- I: Schmelzen von A bei 60°C, dann Zugabe von B
- II: Erwärmen von C auf 60°C
- III: Einrühren von II in I, bis zum Abkühlen auf Raumtemperatur Rühren
- IV: nacheinander Zugabe der Komponenten D zu III bei 35°C
- V: Zugabe von E und Homogenisieren der Emulsion

### Beispiele 2A, 2B und 2C: Anti-Akne Gel

| | | | |
|---|---|---|---|
| A | Octopirox® | (Clariant) | 0.10 % |
| | Pirocton Olamin | | |
| B | Ethanol | | 25.00 % |
| | Propylen Glycol | | 20.00 % |
| C | Parfüm | | 0.20 % |
| | Nipaguard® DCB | (Clariant) | q.s. |
| | Phenoxyethanol (und) Methyldibromo Glutaronitril | | |
| D | Copolymer aus Beispiel A, B oder C | | 1.30 % |
| E | Allantoin | (Clariant) | 0.10 % |
| | Allantoin | | |
| F | Wasser | | ad 100 % |

### Herstellung:

- I: Lösen von A in B
- II: Zugabe von C zu I
- III: Einrühren von D in II
- IV: Lösen von E in F bei 35°C
- V: Zugabe von IV zu III unter Rühren

### Beispiele 3A, 3B und 3C: Feuchtigkeitsmittel

| | | | |
|---|---|---|---|
| A | Wasser | | ad 100% |
| B | Glycerin | | 5.00% |
| | Bozequat® 4000 | (Clariant) | 0.60% |
| | Polyquaternium-43 | | |
| | Polyglykol 35000 S | (Clariant) | 0.50% |
| | PEG-800 | | |
| | Copolymer aus Beispiel A, B oder C | | 0.60% |
| | Duftstoff | | 0.15% |
| | Nipaguard® MPA | (Clariant) | q.s. |
| | Benzyl Alkohol (und) Methylparaben (und) Propylparaben | | |

### Herstellung:

Nacheinander Einrühren der Komponenten B in A.

### Beispiele 4A, 4B und 4C: Gesichtsgel

| | | | |
|---|---|---|---|
| A | Copolymer aus Beispiel A, B oder C | | 1.50 % |
| B | Wasser | | ad 100 % |
| C | Glycerin | | 4.00 % |
| | Propylenglycol | | 3.00 % |
| | Harnstoff | | 0.50 % |
| | Panthenol | | 0.30 % |
| | Sorbitol | | 0.50 % |
| D | Biobranil wasserlöslich | | 1.00 % |
| | Wasser (Aqua), Propylenglycol, Weizenkleie Extract, | | |
| | Ethoxydiglycol, PEG- 40 hydrogeniertes Rizinusöl, | | |
| | Trideceth-9, Tocopherol | | |
| | Aloe-Vera Gel Konzentrat 10:1 | | 1.00 % |
| | Wasser (Aqua), Aleo Barbadensis Gel | | |
| | Hamamelis: | | 1.00 % |
| | Ethoxydiglycol, Propylene Glycol, Wasser (Aqua), | | |
| | Butylene Glycol Witch Hazel (Hamamelis Virginiana) Extrakt | | |
| | Mango | | 1.00 % |
| | Wasser (Aqua), Ethoxydiglycol, Propylene Glycol, | | |
| | Mango (Mangifera Indica) Extract, Butylene Glycol | | |
| | Hyaluronic Acid | | 0.50 % |
| | Hydroviton 24 | | 2.00 % |
| | Wasser (Aqua), Natriumlactat, Milchsäure, Glycerin, | | |
| | Serine, Sorbitol, EA-Lactat, Harnstoff, Natriumchlorid, | | |
| | Lauryl Diethylendiaminoglycerin, Lauryl Aminopropylglycerin, | | |
| | Allantoin, SD-Alcohol 39-c (Alcohol denat.) | | |
| | Uvinul P 25 | | 3.00% |
| | PEG-25 PABA | | |
| | Nipaguard® CMB 0.15 % | (Clariant) | 0.15 % |
| | Triethylen Glycol, Benzyl Alkohol, Propylen Glycol, | | |
| | Chlorömethylisothiazolinone, Methylisothiazolinone | | |
| E | Aminomethylpropanol | | 0.30 % |

### Herstellung:

- I: Lösen der Komponenten C in B
- II: nacheinander Einrühren der Komponenten D in I
- III: Einstellen des pH-Wertes mit E
- IV: Zugabe von A zu III unter Rühren

### Beispiele 5A, 5B und 5C: Hautpflegemittel ohne Öl

| | | | |
|---|---|---|---|
| A | Wasser | | ad 100% |
| B | Glycerin | | 3.00% |
| | Extrapon® Kamille Spezial | | 1.00% |
| | Bisabolol-Extract | | |
| | Polyglykol 400 | (Clariant) | 4.50% |
| | PEG-8 | | |
| | Polyglykol 1500 S | (Clariant) | 1.50% |
| | PEG-32 | | |
| | Harnstoff; chem. pure | | 2.00% |
| | Copolymer aus Beispiel A, B oder C | | 1.00% |
| | Duftstoff | | 0.15% |
| | Nipaguard® CMB | (Clariant) | 0.10 % |
| | Triethylenglykol und Benzylalkohol und Propylenglycol und Chloromethylisothiazolinon und Methylisothiazolinon | | |
| C | NaOH (20 %) | | q.s. |

### Herstellung:

- I: nacheinander Einrühren der Komponenten B in A
- II: Einstellen des pH-Wertes mit C

### Beispiele 6A, 6B und 6C: O/W-Hautmilch

| | | | |
|---|---|---|---|
| A | Hostacerin® DGL | (Clariant) | 2.00 % |
| | Polyglyceryl-2 PEG-10 Laurat | | |
| | Isopropyl Palmitat | | 4.00 % |
| | Almond Öl | | 5.00 % |
| | Weizenkeimöl | | 1.00 % |
| | Cetearylisononanoat | | 8.00 % |
| B | Copolymer aus Beispiel A, B oder C | | 0.80 % |
| C | Wasser | | ad 100 % |
| | Konservierungsmittel | | q. s. |
| D | Duftstoff | | 0.30 % |

### Herstellung:

- I: Mischen von A und B, dann Einrühren in C
- II: Zugabe von D zu I unter Rühren
- III: Homogenisieren der Emulsion

### Beispiele 7A, 7B und 7C: O/W-Creme

| | | | |
|---|---|---|---|
| A | Hostacerin® DGI | (Clariant) | 2.00 % |
| | Polyglyceryl-2 Sesquiisostearat | | |
| | Isopropyl Palmitate | | 4.00 % |
| | Octyldodecanol | | 4.00 % |
| | Nipaguard® PDU | (Clariant) | q.s. |
| | Propylenglycol (und) Diazolidinyl Harnstoff (und) Methylparaben (und) Propylparaben | | |
| B | Copolymer aus Beispiel A, B oder C | | 1.20 % |
| C | Hostapon® CCG | (Clariant) | 0.80 % |
| | Natriumcocoylglutamat | | |
| | Wasser | | ad 100 % |
| D | Duftstoff | | 0.40 % |

### Herstellung:

- I: Einrühren von B in A, dann Zugabe von C und Rühren
- II: Einrühren von D in I
- III: Homogenisieren der Emulsion

### Beispiele 8A, 8B und 8C: O/W Tagescreme mit UVA- und UVB-Lichtschutz

| | | | |
|---|---|---|---|
| A | Hostaphat® KW 340 D | (Clariant) | 1.0 % |
| | Triceteareth-4 Phosphat | | |
| | Glyceryl Stearate | | 0.5 % |
| | Cetearyl Alkohol | | 0.5 % |
| | Mineralöl, niedrigviskos | | 7.0 % |
| | Isopropylpalmitat | | 6.0 % |
| | SilCare® Silicone 41 M15 | (Clariant) | 1.0 % |
| | Caprylyl Methicone | | |
| | Caprylic/Capric Triglyceride | | 2.0 % |
| | Benzophenone-3 | | 1.0 % |
| B | Copolymer aus Beispiel A, B oder C | | 1.0 % |
| C | Wasser | | ad 100 % |
| | Glycerin | | 5.0 % |
| | Tinosorb® M | | 3.0 % |
| | Methylene Bis-Benzotriazolyl Tetramethylbutylphenol | | |
| | Allantoin | (Clariant) | 0.3 % |
| | Allantoin | | |
| D | Tocopheryl Acetate | | 1.0 % |
| | Duftstoff | | 0.3 % |
| | Nipaguard® PDU | (Clariant) | q.s. |
| | Propylenglycol (und) Diazolidinyl Harnstoff (und) | | |
| | Methylparaben (und) Propylparaben | | |

### Herstellung:

- I: Schmelzen von A bei ca. 70°C
- II: Erwärmen von C auf ca. 70°C
- III: Einrühren von B in A, sofort danach Zugabe von C und Rühren bis zum Abkühlen auf ca. 30°C
- IV: Einrühren von D in III bei ca. 30°C

### Beispiele 9A, 9B und 9C: After Sun Cremegel

| | | | |
|---|---|---|---|
| A | Mineralöl, niedrigviskos | | 3.00 % |
| | Isopropyl Palmitat | | 3.00 % |
| | Cetiol® SN | | 3.00 % |
| | Cetearyl Isononanoat | | |
| | Jojoba Öl | | 3.00 % |
| | Wainuss Öl | | 3.00 % |
| | Panthenol | | 1.00 % |
| | Tocopheryl Acetat | | 1.00 % |
| B | Copolymer aus Beispiel A, B oder C | | 1.00 % |
| C | Wasser | | ad 100 % |
| | Glycerin | | 3.00 % |
| | Allantoin | (Clariant) | 0.20 % |
| | Allantoin | | |
| | Konservierungsmittel | | q. s. |
| D | Collagen nativ 1 % | | 3.00 % |
| | Alkohol | | 1.50 % |
| | Duftstoff | | 0.30 % |

### Herstellung:

- 1: Schmelzen von A bei ca. 70°C, dann Zugabe von B
- II: Erwärmen von C auf ca. 70°C
- III: Einrühren von II in I und Abkühlen auf ca. 35°C unter Rühren
- IV: Zugabe von D in III bei ca. 35°C
- V: Homogenisieren der Emulsion

### Beispiele 10A, 10B und 10C: O/W-Sonnenschutzmilch

| | | | |
|---|---|---|---|
| A | Arisfoflex® PEA | (Clariant) | 2.00% |
| | Polypropylene Terephthalate | | |
| | Isopropyl Palmitate | | 5.00 % |
| | Mineralöl, perliquidium | | 10.00 % |
| | Neo Heliopan® E 1000 | | 8.50 % |
| | Isoamyl p-Methoxycinnamate | | |
| | Neo Heliopan® BB | | 1.50 % |
| | Benzophenone-3 | | |
| B | Copolymer aus Beispiel A, B oder C | | 0.60 % |
| C | Wasser | | ad 100 % |
| | Glycerin | | 3.00 % |
| | Konservierungsmittel | | q. s. |
| D | Duftstoff | | 0.30 % |

### Herstellung:

- I: Schmelzen von A bei ca. 60°C, Zugabe von B
- II: Zugabe von C, kräftiges Rühren
- III: Zugabe von D zu II bei ca. 35°C
- IV: Homogenisieren der Emulsion

### Beispiele 11A, 11B und 11C: O/W Sonnenschutzlotion, sprühbar

| | | | |
|---|---|---|---|
| A | Hostacerin® DGI | (Clariant) | 4.00% |
| | Polyglyceryl-2 Sesquiisostearat | | |
| | Eutanol® G | | 4.50% |
| | Octyldodecanol | | |
| | Eusolex® 2292 | | 10.00% |
| | Ethylhexyl Methoxycinnamat | | |
| | Eusolex® 9020 | | 5.00% |
| | Butyl Methoxydibenzoylmethan | | |
| | Eusolex® 6300 | | 4.00% |
| | 4-Methylbenzyliden Camphor | | |
| B | Copolymer aus Beispiel A, B oder C | | 0.50 % |
| C | Wasser | | 40.00% |
| D | Wasser | | ad 100 % |
| | Hostapon® KCG | (Clariant) | 1.50 % |
| | Natriumcocoylglutamate | | |
| | Glycerin | | 3.00 % |
| | Panthenol | | 0.50 % |
| E | Alcohol | | 5.00 % |
| | Tocopheryl Acetat | | 0.50 % |
| | Duftstoff | | q.s. |
| | Nipaguard® PDU | (Clariant) | q.s. |
| | Propylene Glycol (und) Diazolidinyl Harnstoff (und) | | |
| | Methylparaben (und) Propylparaben | | |

### Herstellung:

- I: Schmelzen von A bei ca. 80°C
- II: Zugabe von B zu I
- III: Erwärmen von C auf ca. 80°C
- IV: Einrühren von III in I unter starkem Rühren (Ultraturrax / staro), ca. 2 Minuten
- V: sehr langsames Einrühren einer kalten Lösung von D in IV
- VI: Einrühren von E in V und Rühren, 1 Stunde
- VII: Homogenisieren der Emulsion

### Beispiele 12A, 12B und 12C: Sonnenschutzcreme

| | | | |
|---|---|---|---|
| A | Hostaphat® KL 340 D | (Clariant) | 1.00 % |
| | Trilaureth-4 Phosphat | | |
| | Mineralöl, niedrigviskos | | 8.00 % |
| | Isopropyl Palmitat | | 3.00 % |
| | Myritol 318 | | 2.00 % |
| | Caprylic/Capric Triglycerid | | |
| | Glycerylstearat | | 0.50 % |
| | Cetearylalkohol | | 0.50 % |
| B | Copolymer aus Beispiel A, B oder C | | 0.80 % |
| C | Glycerin | | 5.00 % |
| | Alkohol | | 1.00 % |
| | Wasser | | ad 100 % |
| D | Tocopheryl Acetat | | 1.00 % |
| | Z-Cote HP1 | | 10.00 % |
| | Zinkoxid und Dimethicon | | |
| | Konservierungsmittel | | q. s. |
| E | Duftstoff | | 0.30 % |

### Herstellung:

- I: Schmelzen von A bei ca. 70°C, dann Zugabe von B
- II: Erwärmen von C auf ca. 40°C
- III: Einrühren von II in I

- IV: Zugabe von D zu III bei ca. 35°C
- V: Zugabe von C zu IV

### Beispiele 13A, 13B und 13C: Feuchtigkeitscreme-Gel

| | | | |
|---|---|---|---|
| A | Mineralöl, niedrigviskos | | 6.00 % |
| | Isopropyl Palmitat | | 3.60 % |
| | Sojaöl | | 2.40% |
| B | Copolymer aus Beispiel A, B oder C | | 1.00 % |
| C | Wasser | | ad 100 % |
| | Glycerin | | 8.00% |
| | Nipaguard® PDU | (Clariant) | q.s. |
| | Propylenglycol (und) Diazolidinylharnstoff (und) Methylparaben (und) Propylparaben | | |
| D | Duftstoff | | 0.30 % |

### Herstellung:

- I: Mischen von A und B
- II: Einrühren von C in l, dann Zugabe von D
- III: Homogenisieren der Emulsion

### Beispiele 14A, 14B und 14C: O/W-Anti-Akne Hautmilch

| | | | |
|---|---|---|---|
| A | Hostacerin® DGL | (Clariant) | 1.00 % |
| | PEG-10 Polyglyceryl-2 Laurat | | |
| | Hostacerin® DGSB | (Clariant) | 4.00 % |
| | PEG-4 Polyglyceryl-2 Stearat | | |
| | Mineralöl, niedrigviskos | | 5.00 % |
| | Eutanol® G | (Henkel) | 8.00 % |
| | Octyldodecanol | | |
| | Isopropyl Palmitate | (Unigema) | 5.00 % |
| | Jojoba oil | | 2.00 % |
| B | Copolymer aus Beispiel A, B oder C | | 0.90 % |
| C | Octopirox® | (Clariant) | 0.20 % |
| | Piroctone Olamine | | |
| D | 1,2-Propylenglycol | | 10.00 % |
| E | Wasser | | ad 100 % |
| | Allantoin | (Clariant) | 0.20 % |
| F | Konservierungsmittel | | q.s. |
| | Perfume | | 0.30 % |

### Herstellung:

- I: Schmelzen von A bei ca. 60°C, dann Zugabe von B
- II: Lösen von C in erwärmten D
- III: Einrühren von II in I
- IV: Erwärmen von E auf 60°C
- V: Einrühren von IV in III
- VI: Rühren und auf 35°C Abkühlen
- VII: Zugabe von F zu VI bei ca. 35°C
- VIII: Homogenisieren

### Beispiele 15A, 15B und 15C: O/W-Körper-Lotion

| | | | |
|---|---|---|---|
| A | Mineralöl, niedrigviskos | | 4.00 % |
| | Isopropylpalmitat | | 4.00 % |
| | Eutanol®G | | 4.00 % |
| | Ethylhexyldodecanol | | |
| | Cetiol®HE | | 0.50 % |
| | PEG-7 Glycerylcocoat | | |
| | Cetiol® LC | | 0.50 % |
| | Coco-Caprylate/Caprate | | |
| | SilCare Silicone®41 M15 | (Clariant) | 0.50% |
| | Caprylyl Methicone | | |
| B | Wasser | | ad 100 % |

| | | | |
|---|---|---|---|
| | Glycerin | | 3.00% |
| | Panthenol | | 0.50 % |
| | Polyglykol 35000 S | (Clariant) | 1.50 % |
| | PEG-800 | | |
| | Allantoin | (Clariant) | 0.20 % |
| | Allantoin | | |
| | Copolymer aus Beispiel A, B oder C | | 0.30 % |
| C | Hostapon® KCG | (Clariant) | 6.00 % |
| | Natriumcocoylglutamat | | |
| | Duftstoff . | | 0.30 % |
| D | Nipaguard® PDU | (Clariant) | q.s. |
| | Propylenglycol (und) Dizolidinylharnostoff (und) | | |
| | Methylparaben (und) Propylparben | | |

### Herstellung:

- I: Mischen der Komponenten A
- II: Lösen von B unter Rühren
- III: Zugabe von II to I und kräftiges nach ca. 5 Minuten Zugabe von C
- IV: nach 5 Minuten Zugabe von D

### Beispiele 16A, 16B und 16C: Mascara

| | | | |
|---|---|---|---|
| A | Tylose® H 4000 G4 | (Clariant) | 0.70 % |
| | Hydroxyethylcellulose | | |
| | 1,2-Propyleneglycol | | 1.00 % |
| | Wasser | | ad 100 % |
| B | Triethanolamin 99 % | | 1.20 % |
| C | Stearic Acid | | 3.00 % |
| | SilCare® Silicone 41 M15 | (Clariant) | 1.00 % |
| | Caprylyl Methicon | | |
| | SiICare® Silicone 31 M40 | (Clariant) | 2.00 % |
| | Caprylyl Trimethicon | | |
| | Tegocare®450 | | 4.00 % |
| | Polyglycery-3 Methylglucose Distearat | | |
| | Nexbase® 2006 | | 2.00 % |
| | Poly-1-Decen | | |
| | Bienenwachs | | 2.50 % |
| | Candelilla Wax | | 2.50 % |
| | Lunacera M | | 3.50 % |
| | Mikrowachs | | |
| D | Pigmente | | 10.00 % |
| E | Copolymer aus Beispiel A, B oder C | | 0.40 % |
| F | Nipagin® M (Clariant) | | 0.20% |
| | Methylparaben | | |
| | Nipasol®M | (Clariant) | 0,10 % |
| | Propylparaben | | |
| G | Duftstoff | | q.s./optional |

### Herstellung:

- I: Erwärmen einer Lösung von Tylose H 4000 G4 und Wasser auf 85°C
- II: Zugabe von B zu I unter kräftigem Rühren
- III: Mischen der Komponenten D und Schmelzen bei ca. 85°C
- IV: Zugabe von D und E zu lll, Homogenisieren bei ca. 85°C
- V: Zugabe von II zu IV und kräftiges Rühren, ca. 30 Minuten
- VI: Zugabe von F zu IV bei ca. 35°-40°C.

### Beispiele 17A, 17B und 17C: Haargel

| | | | |
|---|---|---|---|
| A | Genapol® HS 200 | (Clariant) | 0.20 % |
| | Steareth-20 | | |
| | Duftstoff | | 0.20 % |
| B | Wasser | | ad 100 % |
| | Genapol® HS 200 | (Clariant) | 1.80 % |
| | Steareth 20 | | |
| C | Propylenglycol | | 2.00 % |
| | Diaformer® Z-651 | (Clariant) | 4.50 % |
| | Acrylate/Lauryl Acrylat/Stearyl Acrylat/Ethylaminoxid | | |
| | Methacrylat Copolymer | | |
| | Alkohol denat. | | 20.00 % |
| D | Farbstoff | | q.s. |
| | Nipaguard® MPA | (Clariant) | 0.50 % |
| | Benzyl Alkohol (und) Methylparaben (und) Propylparaben | | |
| E | Copolymer aus Beispiel A, B oder C | | 0.80 % |

### Herstellung:

- 1: Mischen der Komponenten A
- II: Lösen der Komponenten B unter mildem Erwärmen. Abkühlen und zu I geben
- III: nacheinander Zugabe der Komponenten C zu II
- IV: Zugabe der Komponenten D zu III
- V: Zugabe von E zu IV und Homogenisieren

### Beispiele 18A, 18B und 18C: Styling Fluid

| | | | |
|---|---|---|---|
| A | Sorbitol | | 5.00% |
| | Bozequat® 4000. | (Clariant) | 0.30% |
| | Polyquaternium-43 | | |
| B | Wasser | | ad 100 % |
| C | Copolymer aus Beispiel A, B oder C | | 2.00 % |
| D | Aristoflex® A 60 | (Clariant) | 5.00 % |
| | VA/Crotonates Copolymer | | |
| | Emulsogen® HCO 040 | (Clariant) | 4.00 % |
| | PEG-40 hydriertes Rizinusöl | | |
| E | Duftstoff | | 0.20 % |
| | Nipaguard® DMDMH | (Clariant) | 0.30 % |
| | DMDM Hydantoin | | |
| | Farbstoff | | q.s. |
| | Timiron Diamond Cluster MP-149 | | q.s. |
| | Glimmer und Titandioxid EU: Cl 77891) | | |
| F | Aminomethyl Propanol | | 0.30 % |

### Herstellung:

- I: Mischen der Komponenten A
- II: Zugabe von B zu I
- III: Aufquellen von C in 11 unter Rühren
- IV: Mischen der Komponenten D und Zugabe von IV zu 111
- V: nacheinander Zugabe der Komponenten von E zu IV
- VI: Einstellen auf pH 7.0 mit F

### Beispiele 19A, 19B und 19C: Nagellackentferner, Gel

| | | | |
|---|---|---|---|
| A | Copolymer aus Beispiel A, B oder C | | 1.50 % |
| B | Wasser | | ad 100 % |
| C | Aceton | | 50.00 % |
| | Glycerin | | 5.00 % |
| | Polyglykol 400 | (Clariant) | 3.00 % |
| | PEG-8 | | |

### Herstellung:

- I: nacheinander Lösen der Komponenten C in B
- II: Einrühren von A in I

### Beispiele 20A, 20B und 20C: Duschbad mit Glittereffekt

| | | | |
|---|---|---|---|
| A | Wasser | | ad 100 % |
| B | Copolymer aus Beispiel A, B oder C | | 1.50 % |
| C | Genapol® LRO flüssig | (Clariant) | 30.00 % |
| | Natriumlaurethsulfat | | |
| | Duftstoff | | 0.50 % |
| | Nipaguard® DCB | (Clariant) | 0.10 % |
| | Phenoxyethanol (und) Methyldibromo Glutaronitril | | |
| D | Hostapon® KCG | (Clariant) | 5.00 % |
| | Natriumcocoylglutamat | | |
| E | Cirebelle 104 | | 0.20 % |
| | Synthetisches Wachs | | |

### Herstellung:

- I: Zugabe von A zu B und Rühren zu einem homogenen Gel
- II: Mischen der Komponenten C und Zugabe zu I unter Rühren, Homogenisieren
- III: Zugabe von D zu II
- IV: Zugabe von E zu III
- V: Einstellen des pH-Wertes

### Beispiele 21A, 21B und 21C: Gesichtreinigungslotion mit pflegender Wirkung

| | | | |
|---|---|---|---|
| A | Glycerin | | 8.00 % |
| | Polyglykol 400. | (Clariant) | 5.00 % |
| | PEG-8 | | |
| | Panthenol | | 0.50 % |
| | Duftstoff | | 0.20 % |
| | Alkohol | | 8.00 % |
| | Konservierungsmittel | | q.s. |
| | Allantoin | (Clariant) | 0.10 % |
| | Allantoin | | |
| | Niacinamid | | 0.10 % |
| | Extrapon Hamamelis | | 1.00 % |
| | Wasser, Quecke-Nussdestillat, SD Alkohol 39-C, Butylenglycol | | |
| B | Wasser | | ad 100 % |
| C | Copolymer aus Beispiel A, B oder C | | 0.30 % |

### Herstellung:

- 1: Lösen von A in B unter Rühren
- II: Zugabe von C zu I unter Rühren und Homogenisieren

### Beispiele 22A, 22B und 22C: erfrischendes Körpergel mit konditionierender Wirkung

| | | | |
|---|---|---|---|
| A | Wasser | | ad 100 % |
| B | Alkohol denat. | | 20.00 % |
| | Glycerin | | 5.00 % |
| | Polyglykol 35000 S | (Clariant) | 0.50 % |
| | PEG-800 | | |
| | Allantoin | (Clariant) | 0.20 % |
| | Allantoin | | |
| | Copolymer aus Beispiel A, B oder C | | 1.00 % |
| | Duftstoff | | 0.15% |
| | Nipaguard® MPA | (Clariant) | q.s. |
| | Benzylalkohol (und) Methylparaben (und) Propylparaben | | |

### Herstellung:

Nacheinander Einrühren der Komponenten B in A

### Beispiele 23A, 23B und 23C: klares Sonnenschutzmittelgel

| | | | |
|---|---|---|---|
| A | Ethanol | | 50.00 % |
| | Wasser | | ad 100 % |
| | Uvinul P25 | | 6.00 % |
| | PEG-25 PABA | | |
| | Glycerin | | 4.00 % |
| | D-Panthenol | | 1.00 % |
| | Panthenol | | |
| | Dow Corning 193 Tensid | | 0.50 % |
| | PEG-12 Dimethicone | | |
| B | Copolymer aus Beispiel A, B oder C | | 1.00 % |
| C | Duftstoff | | 0.30 % |

### Herstellung:

- I: Einrühren von B in A
- II: Zugabe von C

### Beispiele 24A, 24B und 24C: O/W Sonnenschutzmittel, sprühbarer Schaum

| | | | |
|---|---|---|---|
| A | Eusolex® 2292 | | 8.00 % |
| | Ethylhexylmethoxycinnamat | | |
| | Eusolex® HMS | | 8.00 % |
| | Homosalat | | |
| | Eusolex® 9020 | | 4.00 % |
| | Butylmethoxydibenzoylmethan | | |
| | Eusolex® 6300 | | 4.00% |
| | 4-Methylbenzyliden Camphor | | |
| | Isopropylpalmitat | | 2.00 % |
| | SilCare Silicone® 41 M15 | (Clariant) | 0.50 % |
| | Caprylyl Methicone | | |
| | Eutanol® G | | 2:00 % |
| | Octyldodecanol | | |
| B | wasser | | ad 100% |
| | Glycerin | | 7.00% |
| | Panthenol | | 0.50 % |
| | Polyglykol 35000 S | (Clariant) | 2.00 % |
| | PEG-800 | | |
| | Copolymer aus Beispiel A, B oder C | | 0.40 % |
| C | Hostapon® KCG | (Clariant) | 12.00 % |
| | Natriumcocoylglutamat | | |
| | Duftstoff | | 0.30 % |
| D | Nipa Konservierungsmittel | (Clariant) | q.s. |

### Herstellung:

- I: Mischen der Komponenten A, Erwärmen auf 70°C
- II: Lösen von B unter Rühren und Erwärmen auf 70°C
- III: Zugabe von II zu I und kräftiges Rühren
- IV: Zugabe von C bei ca. 35°C
- V: Nach 5 Minuten Zugabe von D

### Beispiele 25A, 25B und 25C: Handwaschgel

| | | |
|---|---|---|
| A | Copolymer aus Beispiel A, B oder C | 0.80 % |
| | Isopropyl Alkohol | 50.60 % |
| B | Wasser | ad 100 % |
| C | Imwitor® 312 | 2.00 % |
| | Glyceryllaurat | |
| | Propylenglycol | 2.00 % |
| | Glycerin | 2.00 % |
| D | Farbstoff | q.s. |

### Herstellung:

- I: Lösen der Komponenten of A in B unter Rühren
- II: Zugabe der Komponenten C zu I
- III: Zugabe von D zu II

## Patentansprüche

1. Kosmetisches, dermatologisches und/oder pharmazeutisches Mittel, **dadurch gekennzeichnet, dass** es mindestens ein Copolymer **A,** enthaltend
a) 1 bis 50 Gew.-% an Struktureinheiten hervorgegangen aus N-Vinylcaprolactam,
b) 49,99 bis 98,99 Gew.-% an wiederkehrenden Struktureinheiten der Formel (1) 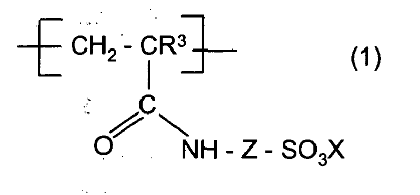 worin
R³ Wasserstoff, Methyl oder Ethyl,
Z C₁-C₈-Alkylen und
X⁺ Li⁺, Na⁺, K⁺, Mg²⁺/2, Ca²⁺/2, Al³⁺/3, NH₄⁺, Monoalkylammonium, Dialkylammonium, Trialkylammonium oder Tetraalkylammonium, wobei es sich bei den Alkylsubstituenten der Ammmoniumionen unabhängig voneinander um (C₁-C₂₂)-Alkylreste oder (C₂-C₁₀)-Hydroxyalkylreste handelt, oder ein- bis dreifach ethoxylierte Ammoniumverbindungen mit gleichem oder unterschiedlichem Ethoxylierungsgrad bedeutet,
und wobei auch mehrere unterschiedliche Struktureinheiten der Formel (1) in dem Copolymer enthalten sein können, und
c) 0,01 bis 8 Gew.-%, vorzugsweise 0,01 bis 5 Gew.-% an vernetzenden Strukturen, die aus Monomeren mit mindestens zwei olefinischen Doppelbindungen hervorgegangen sind,
enthält.

2. Mittel nach Anspruch 1, **dadurch gekennzeichnet, dass** die Copolymere **A** 2 bis 30 Gew.-%, vorzugsweise 3 bis 15 Gew.-% an Struktureinheiten hervorgegangen aus N-Vinylcaprolactam, 69,5 bis 97,5 Gew.-%, vorzugsweise 84,5 bis 96,5 Gew.-% an Struktureinheiten der allgemeinen Formel (1), insbesondere abgeleitet vom Ammoniumsalz der 2-Acrylamido-2-methyl-propan-sulfonsäure und 0,2 bis 4 Gew.-%, vorzugsweise 0,5 bis 3 Gew.-% an vernetzenden Strukturen, die aus Monomeren mit mindestens zwei olefinischen Doppelbindungen hervorgegangen sind, enthalten.

3. Mittel nach Anspruch 1 oder 2, **dadurch gekennzeichnet, dass** es sich bei den vernetzenden Strukturen um Strukturen hervorgegangen aus Trimethylolpropantriacrylat handelt.

4. Mittel nach einem oder mehreren der Ansprüche 1 bis 3, **dadurch gekennzeichnet, dass** es, bezogen auf das Gesamtgewicht des Mittels, 0,01 bis 10 Gew.-%, vorzugsweise 0,1 bis 5 Gew.-%, besonders bevorzugt 0,5 bis 3 Gew.-% der Copolymere **A** enthält.

5. Mittel nach einem oder mehreren der Ansprüche 1 bis 4, **dadurch gekennzeichnet, dass** es, bezogen auf das Gesamtgewicht des Mittels, 0,01 bis 20 Gew.-%, vorzugsweise 0,5 bis 10 Gew.-%, besonders bevorzugt 1 bis 5 Gew.-% an Hydroxysäuren, vorzugsweise α-Hydroxysäuren, die auch teilweise als Salz vorliegen können, enthält.

6. Mittel nach einem oder mehreren der Ansprüche 1 bis 5, **dadurch gekennzeichnet, dass** es Glykolsäure, Milchsäure und/oder 2-Hydroxyoctansäure enthält.

7. Mittel nach einem oder mehreren der Ansprüche 1 bis 6, **dadurch gekennzeichnet, dass** es als Emulsion vom Typ Öl-in-Wasser vorliegt, und bezogen auf das Gesamtgewicht des Mittels
a) bis zu 95 Gew.-%, vorzugsweise 60 bis 92 Gew.-%, besonders bevorzugt 70 bis 90 Gew.-%, insbesondere bevorzugt 75 bis 85 Gew.-% einer Wasserphase,
b) bis zu 40 Gew.-%, vorzugsweise 1 bis 40 Gew.-%, besonders bevorzugt 2 bis 25 Gew.-%, insbesondere bevorzugt 5 bis 20 Gew.-% einer Ölphase,
c) bis zu 15 Gew.-%, vorzugsweise 0,5 bis 12 Gew.-%, besonders bevorzugt 1 bis 8 Gew.-%, insbesondere bevorzugt 1 bis 5 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymer **A**
enthält.

8. Mittel nach einem oder mehreren der Ansprüche 1 bis 6, **dadurch gekennzeichnet, dass** es als Gelcreme vom Typ Öl-in-Wasser vorliegt, und bezogen auf das Gesamtgewicht des Mittels
a) bis zu 95 Gew.-%, vorzugsweise 50 bis 95 Gew.-%, besonders bevorzugt 70 bis 90 Gew.-%, insbesondere bevorzugt 75 bis 85 Gew.-% einer Wasserphase,
b) bis zu 30 Gew.-%, vorzugsweise 1 bis 30 Gew.-%, besonders bevorzugt 3 bis 25 Gew.-%, insbesondere bevorzugt 5 bis 15 Gew.-% einer Ölphase,
c) bis zu 5 Gew.-%, vorzugsweise 0,5 bis 5 Gew.-%, besonders bevorzugt 0,2 bis 4 Gew.-%, insbesondere bevorzugt 0,5 bis 3 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymer **A**
enthält.

9. Mittel nach einem oder mehreren der Ansprüche 1 bis 6, **dadurch gekennzeichnet, dass** es als Emulsion vom Typ Wasser-in-Öl vorliegt, und bezogen auf das Gesamtgewicht des Mittels
a) bis zu 95 Gew.-%, vorzugsweise 40 bis 95 Gew.-%, besonders bevorzugt 50 bis 90 Gew.-%, insbesondere bevorzugt 60 bis 85 Gew.-% einer Wasserphase,
b) bis zu 60 Gew.-%, vorzugsweise 2 bis 60 Gew.-%, besonders bevorzugt 5 bis 40 Gew.-%, insbesondere bevorzugt 10 bis 30 Gew.-% einer Ölphase,
c) bis zu 20 Gew.-%, vorzugsweise 0,5 bis 20 Gew.-%, besonders bevorzugt 1 bis 15 Gew.-%, insbesondere bevorzugt 4 bis 12 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymer **A** enthält.

10. Mittel nach einem oder mehreren der Ansprüche 1 bis 6, **dadurch gekennzeichnet, dass** es als Wasser-in-Silikon-Emulsion vorliegt und bezogen auf das Gesamtgewicht des Mittels
a) bis zu 90 Gew.-%, vorzugsweise 20 bis 90 Gew.-%, besonders bevorzugt 40 bis 85 Gew.-%, insbesondere bevorzugt 60 bis 80 Gew.-% einer Wasserphase,
b) bis 80 Gew.-%, vorzugsweise 10 bis 70 Gew.-%, besonders bevorzugt 20 bis 60 Gew.-%, insbesondere bevorzugt 30 bis 50 Gew.-% an Silikonöl,
c) 0,5 bis 20 Gew.-%, vorzugsweise 1 bis 15 Gew.-%, besonders bevorzugt 3 bis 10 Gew.-% eines oder mehrerer Emulgatoren und
d) 0,01 bis 5 Gew.-%, vorzugsweise 0,05 bis 3 Gew.-%, besonders bevorzugt 0,1 bis 2 Gew.-% an Copolymer **A**
enthält.

11. Mittel nach einem oder mehreren der Ansprüche 1 bis 6, **dadurch gekennzeichnet, dass** es als Wasser-in-Silikon-Emulsionen vorliegt und bezogen auf das Gesamtgewicht des Mittels
a) bis zu 90 Gew.-%, vorzugsweise 20 bis 90 Gew.-%, besonders bevorzugt 40 bis 85 Gew.-%, insbesondere bevorzugt 60 bis 80 Gew.-% einer Wasserphase,
b) bis 80 Gew.-%, vorzugsweise 10 bis 70 Gew.-%, besonders bevorzugt 20 bis 60 Gew.-%, insbesondere bevorzugt 30 bis 50 Gew.-% an Silikonöl,
c) 0,5 bis 20 Gew.-%, vorzugsweise 1 bis 15 Gew.-%, besonders bevorzugt 3 bis 10 Gew.-% eines oder mehrerer Emulgatoren ausgewählt aus der Gruppe Cetyldimethiconecopolyol, Lauryldimethiconcopolyol, PEG/PPG-18/18 Dimethicon, Trimethylsilylamodimethicon und
d) 0,01 bis 5 Gew.-%, vorzugsweise 0,05 bis 3 Gew.-%, besonders bevorzugt 0,1 bis 2 Gew.-% an Copolymer **A**
enthält.

12. Mittel nach einem oder mehreren der Ansprüche 1 bis 11, **dadurch gekennzeichnet, dass** es eine oder mehrere UV-Filtersubstanzen enthält.

13. Mittel nach einem oder mehreren der Ansprüche 1 bis 12, **dadurch gekennzeichnet, dass** es ein oder mehrere Hautbefeuchtungsmittel und/oder Farbstoffe und/oder farbgebende Pigmente enthält.

14. Mittel nach einem oder mehreren der Ansprüche 1 bis 6, **dadurch gekennzeichnet, dass** es in Form einer Suspension vorliegt und bezogen auf das Gesamtgewicht des Mittels
a) 0,1 bis 10 Gew.-%, vorzugsweise 0,2 bis 6 Gew.-%, besonders bevorzugt 0,3 bis 5 Gew.-% an Copolymer **A** und
b) 0,1 bis 30 Gew.-%, vorzugsweise 0,5 bis 15 Gew.-%, besonders bevorzugt 1,0 bis 10 Gew.-% an festen Partikeln, insbesondere ausgewählt aus der Gruppe der Farbstoffe, farbgebenden Pigmente, Effekt- und Lichtschutzpigmente, Adsorbentien und Abrasivkomponenten
enthält.

15. Mittel nach Anspruch 14, **dadurch gekennzeichnet, dass** es als Lidschatten auf Gelbasis vorliegt und bezogen auf das Gesamtgewicht des Mittels
a) 0,1 bis 10 Gew.-%, vorzugsweise 0,2 bis 6 Gew.-%, besonders bevorzugt 0,3 bis 5 Gew.-% an Copolymer **A** und
b) 0,1 bis 30 Gew.-%, vorzugsweise 0,5 bis 15 Gew.-%, besonders bevorzugt 1,0 bis 10 Gew.-% an Farbstoffen und/oder farbgebenden Pigmenten
enthält.

16. Mittel nach einem oder mehreren der Ansprüche 1 bis 15, **dadurch gekennzeichnet, dass** es als Haarfärbe-, Haarbleich- und/oder -tönungsmittel vorliegt und eine oder mehrere Substanzen ausgewählt aus Direktfarbstoffen, Oxidationsfarbstoffvorstufen und Bleichmitteln enthält.

17. Mittel nach einem oder mehreren der Ansprüche 1 bis 15, **dadurch gekennzeichnet, dass** es als Haarbleichmittel vorliegt und ein oder mehrere Oxidationsmittel, vorzugsweise Wasserstoffperoxid, enthält.

18. Mittel nach einem oder mehreren der Ansprüche ,1 bis 15, **dadurch gekennzeichnet, dass** es als Deodorant oder Antiperspirant vorliegt.

19. Mittel nach einem oder mehreren der Ansprüche 1 bis 15, **dadurch gekennzeichnet, dass** es als Haarfärbe-, -bleich-, -pflege-, oder ―stylingmittel vorliegt.

20. Mittel nach einem oder mehreren der Ansprüche 1 bis 19, **dadurch gekennzeichnet, dass** es in einer pumpbaren, sprühbaren und/oder verschäumbaren Form vorliegt.

21. Mittel nach Anspruch 20, **dadurch gekennzeichnet, dass** es kein Aerosol enthält.
